# EUROPEAN PATENT SPECIFICATION

(11) **EP 0 773 441 B1**
(45) Date of publication and mention of the grant of the patent: **02.08.2000**
(21) Application number: 96250195.3
(22) Date of filing: 10.09.1996
(51) Int. Cl.: G01N 33/567, G01N 33/50, C07K 1/04, A61K 47/48

(54) **Molecules that home to a selected organ or tissue in vivo and methods of identifying same**
Moleküle, die sich in ausgewählten Organen oder Geweben invivo Einfinden und Verfahren zu ihrer Identifizierung
Molécules qui s'adressent in vivo vers un organe ou tissu prélevé et méthodes pour leur identification

(30) Priority: 11.09.1995 US 526708; 11.09.1995 US 526710
(43) Date of publication of application: 14.05.1997
(60) Divisional application: 99250432.4
(73) Proprietor: LA JOLLA CANCER RESEARCH FOUNDATION, La Jolla, CA 92037 (US)
(72) Inventor: Ruoslahti, Erkki I., Rancho Santa Fe, CA 92067 (US); Pasqualini, Renata, Solana Beach, CA 92075 (US)
(74) Representative: UEXKÜLL & STOLBERG

(56) References cited:
- EP-A- 0 135 277
- EP-A- 0 639 584
- WO-A-92/00091
- WO-A-92/03461
- WO-A-92/06191
- WO-A-95/14714
- CHEMICAL ABSTRACTS, vol. 123, no. 11, 11 September 1995 Columbus, Ohio, US; abstract no. 141201, P. CATTANI ET AL.: "Cloning and characterization of human recombinant antibody Fab fragments specific for types 1 and 2 herpes simplex virus." page 1015; column 1; XP002023099 & MICROBIOLOGICA, vol. 18, no. 2, pages 135-142,
- PROCEEDINGS OF THE NATIONAL ACADEMY OF SCIENCES OF USA, vol. 91, WASHINGTON US, pages 355-359, XP002023097 R. BURIONI ET AL.: "Recombinant human Fab to glycoprotein D neutralizes infectivity and prevents cell-to cell transmission of herpes simplex viruses 1 and 2 in vitro."
- PROCEEDINGS OF THE NATIONAL ACADEMY OF SCIENCES OF USA, vol. 91, WASHINGTON US, pages 7129-7133, XP002023098 R. J. GOODSON ET AL.: "High-affinity urokinase receptor antagonists identified with bacteriophage peptide display."

### SEQUENCE LISTING

(1) GENERAL INFORMATION:
   (i) APPLICANT: The Jolla cancer Research Foundation
   (ii) TITLE OF INVENTION: Molecules That Home to a Selected Organ or Tissue In vivo and Methods of Identifying same
   (iii) NUMBER OF SEQUENCES; 59
   (iv) CORRESPONDENCE ADDRESS:
      (A) ADDRESSEE: Campbell & Flores LLP
      (B) STREET: 4370 La Jolla village Drive, Suite 700
      (C) CITY: San Diego
      (D) STATE: California
      (E) COUNTRY: United States
      (F) ZIP: 92122
   (v) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: PatentIn Release #1.0, Version #1.25
   (vi) CURRENT APPLICATION DATA:
      (A) APPLICATION NUMBER:
      (B) FILING DATE:
      (C) CLASSIFICATION:
   (vii) PRIOR APPLICATION DATA:
      (A) APPLICATION NUMBER: US 08/526,710
      (B) FILING DATE: 11-SEP-1995
   (vii) PRIOR APPLICATION DATA:
      (A) APPLICATION NUMBER: US 08/526,708
      (B) FILING DATE: 11-SEP-1995
   (viii) ATTORNEY/AGENT INFORMATION:
      (A) NAME: Campbell, Cathryn A.
      (B) REGISTRATION NUMBER: 31,815
      (C) REFERENCE/DOCKET NUMBER: FP-LJ 2173
   (ix) TELECOMMUNICATION INFORMATION;
      (A) TELEPHONE: (619) 535-9001
      (B) TELEFAX: (619) 535-8949
(2) INFORMATION FOR SEQ ID NO:1:
   (i) SEQUENCE CHARACTERISTICS:
      (A) LENGTH: 9 amino acids
      (B) TYPE: amino acid
      (D) TOPOLOGY: both
   (ii) MOLECULE TYPE: peptide
   (xi) SEQUENCE DESCRIPTION: SEQ ID NO.1:
   Cys Asn Ser Arg Leu His Leu Arg Cys
   1               5
(2) INFORMATION FOR SEQ ID NO:2:
   (i) SEQUENCE CHARACTERISTICS:
      (A) LENGTH: 9 amino acids
      (B) TYPE: amino acid
      (D) TOPOLOGY: both
   (ii) MOLECULE TYPE: peptide
   (xi) SEQUENCE DESCRIPTION: SEQ ID NO:2:
   Cys Glu Asn Trp Trp Gly Asp Val Cys
   1               5
(2) INFORMATION FOR SEQ ID NO:3:
   (i) SEQUENCE CHARACTERISTICS:
      (A) LENGTH: 9 amino acids
      (B) TYPE: amino acid
      (D) TOPOLOGY: both
   (ii) MOLECULE TYPE: peptide
   (xi) SEQUENCE DESCRIPTION: SEQ ID NO:3:
   Cys Leu Ser Ser Arg Leu Asp Ala Cys
   1               5
(2) INFORMATION FOR SEQ ID NO:4:
   (i) SEQUENCE CHARACTERISTICS:
      (A) LENGTH: 8 amino acids
      (B) TYPE: amino acid
      (D) TOPOLOGY: both
   (ii) MOLECULE TYPE: peptide
   (xi) SEQUENCE DESCRIPTION: SEQ ID NO:4:
   Cys Val Leu Arg Gly Gly Arg Cys
   1               5
(2) INFORMATION FOR SEQ ID NO:5:
   (i) SEQUENCE CHARACTERISTICS:
      (A) LENGTH: 9 amino acids
      (B) TYPE: amino acid
      (D) TOPOLOGY: both
   (ii) MOLECULE TYPE: peptide
   (xi) SEQUENCE DESCRIPTION; SEQ ID NO:5:
   Cys Asn Ser Arg Leu Gln Leu Arg Cys
   1 5
(2) INFORMATION FOR SEQ ID NO:6:
   (i) SEQUENCE CHARACTERISTICS:
      (A) LENGTH: 7 amino acids
      (B) TYPE: amino acid
      (D) TOPOLOGY: both
   (ii) MOLECULE TYPE: peptide
   (xi) SEQUENCE DESCRIPTION: SEQ ID NO:6:
   Cys Gly Val Arg Leu Gly Cys
   1 5
(2) INFORMATION FOR SEQ ID NO:7:
   (i) SEQUENCE CHARACTERISTICS:
      (A) LENGTH: 8 amino acids
      (B) TYPE: amino acid
      (D) TOPOLOGY: both
   (ii) MOLECULE TYPE: peptide
   (xi) SEQUENCE DESCRIPTION: SEQ ID No:7:
   Cys Lys Asp Trp Gly Arg Ile Cys
   1 5
(2) INFORMATION FOR SEQ ID NO:8:
   (i) SEQUENCE CHARACTERISTICS:
      (A) LENGTH: 8 amino acids
      (B) TYPE: amino acid
      (D) TOPOLOGY: both
   (ii) MOLECULE TYPE: peptide
   (xi) SEQUENCE DESCRIPTION: SEQ ID NO:8:
   Cys Leu Asp Trp Gly Arg Ile Cys
   1 5
(2) INFORMATION FOR SEQ ID NO:9:
   (i) SEQUENCE CHARACTERISTICS:
      (A) LENGTH: 8 amino acids
      (B) TYPE: amino acid
      (D) TOPOLOGY: both
   (ii) MOLECULE TYPE: peptide
   (xi) SEQUENCE DESCRIPTION: SEQ ID NO:9:
   Cys Thr Arg Ile Thr Glu Ser Cys
   1               5
(2) INFORMATION FOR SEQ ID NO:10:
   (i) SEQUENCE CHARACTERISTICS:
      (A) LENGTH: 7 amino acids
      (B) TYPE: amino acid
      (D) TOPOLOGY: both
   (ii) MOLECULE TYPE: peptide
   (xi) SEQUENCE DESCRIPTION: SEQ ID NO:10:
   Cys Glu Thr Leu Pro Ala Cys
   1               5
(2) INFORMATION FOR SEQ ID NO:11:
   (i) SEQUENCE CHARACTERISTICS:
      (A) LENGTH: 8 amino acids
      (B) TYPE: amino acid
      (D) TOPOLOGY: both
   (ii) MOLECULE TYPE: peptide
   (xi) SEQUENCE DESCRIPTION: SEQ ID NO:11:
   Cys Arg Thr Gly Thr Leu Phe Cys
   1               5
(2) INFORMATION FOR SEQ ID NO:12:
   (i) SEQUENCE CHARACTERISTICS:
      (A) LENGTH: 8 amino acids
      (B) TYPE: amino acid
      (D) TOPOLOGY: both
   (ii) MOLECULE TYPE: peptide
   (Xi) SEQUENCE DESCRIPTION: SEQ ID NO:12:
   Cys Gly Arg Ser Leu Asp Ala Cys
   1 5
(2) INFORMATION FOR SEQ ID NO:13:
   (i) SEQUENCE CHARACTERISTICS:
      (A) LENGTH: 9 amino acids
      (B) TYPE: amino acid
      (D) TOPOLOGY: both
   (ii) MOLECULE TYPE: peptide
   (xi) SEQUENCE DESCRIPTION: SEQ ID NO:13:
   Cys Arg His Trp Phe Asp Val Val Cys
   1 5
(2) INFORMATION FOR SEQ ID NO:14:
   (i) SEQUENCE CHARACTERISTICS;
      (A) LENGTH: 8 amino acids
      (B) TYPE: amino acid
      (D) TOPOLOGY: both
   (ii) MOLECULE TYPE: peptide
   (xi) SEQUENCE DESCRIPTION: SEQ ID NO:14:
   Cys Ala Asn Ala Gln Ser His Cys
   1 5
(2) INFORMATION FOR SEQ ID NO:15:
   (i) SEQUENCE CHARACTERISTICS:
      (A) LENGTH: 8 amino acids
      (B) TYPE: amino acid
      (D) TOPOLOGY: both
   (ii) MOLECULE TYPE: peptide
   (xi) SEQUENCE DESCRIPTION: SEQ ID NO:15:
   Cys Gly Asn Pro Ser Tyr Arg Cys
   1 5
(2) INFORMATION FOR SEQ ID NO:16;
   (i) SEQUENCE CHARACTERISTICS:
      (A) LENGTH: 21 amino acids
      (B) TYPE: amino acid
      (D) TOPOLOGY: both
   (ii) MOLECULE TYPE: peptide
   (xi) SEQUENCE DESCRIPTION: SEQ ID NO:16:
   Trp Arg Cys Val Leu Arg Glu Gly Pro Ala Gly Gly Cys Ala Trp Phe
   1               5                   10                  15
   Asn Arg His Arg Leu
               20
(2) INFORMATION FOR SEQ ID NO:17:
   (i) SEQUENCE CHARACTERISTICS:
      (A) LENGTH: 20 amino acids
      (B) TYPE: amino acid
      (D) TOPOLOGY: both
   (ii) MOLECULE TYPE: peptide
   (xi) SEQUENCE DESCRIPTION: SEQ ID NO:17:
   Tyr Pro Cys Gly Gly Glu Ala Val Ala Gly Val Ser Ser Val Arg Thr
   1               5                   10                  15
   Met Cys Ser Glu
               20
(2) INFORMATION FOR SEQ ID NO:18:
   (i) SEQUENCE CHARACTERISTICS:
      (A) LENGTH: 20 amino acids
      (B) TYPE: amino acid
      (D) TOPOLOGY: both
   (ii) MOLECULE TYPE: peptide
   (xi) SEQUENCE DESCRIPTION: SEQ ID NO:18:
   Leu Asn Cys Asp Tyr Gln Gly Thr Asn Pro Ala Thr Ser Val Ser Val
   1               5                   10                  15
   Pro Cys Thr Val
               20
(2) INFORMATION FOR SEQ ID NO:19:
   (i) SEQUENCE CHARACTERISTICS:
      (A) LENGTH: 7 amino acids
      (B) TYPE: amino acid
      (D) TOPOLOGY: linear
   (ii) MOLECULE TYPE: peptide
   (xi) SEQUENCE DESCRIPTION: SEQ ID NO:19:
   Leu Ser Ser Arg Leu Asp Ala
   1 5
(2) INFORMATION FOR SEQ ID NO:20:
   (i) SEQUENCE CHARACTERISTICS:
      (A) LENGTH: 9 amino acids
      (B) TYPE: amino acid
      (D) TOPOLOGY: linear
   (ii) MOLECULE TYPE: peptide
   (xi) SEQUENCE DESCRIPTION: SEQ ID NO:20:
   Val Leu Arg Glu Gly Pro Ala Gly Gly
   1 5
(2) INFORMATION FOR SEQ ID NO:21:
   (i) SEQUENCE CHARACTERISTICS:
      (A) LENGTH: 7 amino acids
      (B) TYPE: amino acid
      (D) TOPOLOGY: both
   (ii) MOLECULE TYPE: peptide
   (xi) SEQUENCE DESCRIPTION: SEQ ID NO:21:
   Cys Leu Pro Val Ala Ser Cys
   1 5
(2) INFORMATION FOR SEQ ID NO:22:
   (i) SEQUENCE CHARACTERISTICS:
      (A) LENGTH: 7 amino acids
      (B) TYPE: amino acid
      (D) TOPOLOGY: both
   (ii) MOLECULE TYPE: peptide
   (xi) SEQUENCE DESCRIPTION: SEQ ID NO:22:
   Cys Gly Ala Arg Glu Met Cys
   1 5
(2) INFORMATION FOR SEQ ID NO:23.
   (i) SEQUENCE CHARACTERISTICS:
      (A) LENGTH: 8 amino acids
      (B) TYPE: amino acid
      (D) TOPOLOGY: both
   (ii) MOLECULE TYPE: peptide
   (xi) SEQUENCE DESCRIPTION: SEQ ID NO:23:
   Cys Lys Gly Arg Ser Ser Ala Cys
   1               5
(2) INFORMATION FOR SEQ ID NO:24:
   (i) SEQUENCE CHARACTERISTICS:
      (A) LENGTH: 8 amino acids
      (B) TYPE: amino acid
      (D) TOPOLOGY: both
   (ii) MOLECULE TYPE: peptide
   (xi) SEQUENCE DESCRIPTION: SEQ ID NO:24:
   Cys Trp Ala Arg Ala Gln Gly Cys
   1               5
(2) INFORMATION FOR SEQ ID NO:25:
   (i) SEQUENCE CHARACTERISTICS:
      (A) LENGTH: 8 amino acids
      (B) TYPE: amino acid
      (D) TOPOLOGY: both
   (ii) MOLECULE TYPE: peptide
   (xi) SEQUENCE DESCRIPTION: SEQ ID NO:25:
   Cys Leu Gly Arg Ser Ser Val Cys
   1               5
(2) INFORMATION FOR SEQ ID NO:26:
   (i) SEQUENCE CHARACTERISTICS:
      (A) LENGTH: 8 amino acids
      (B) TYPE: amino acid
      (D) TOPOLOGY: both
   (ii) MOLECULE TYPE: peptide
   (xi) SEQUENCE DESCRIPTION: SEQ ID NO:26:
   Cys Thr Ser Pro Gly Gly Ser Cys
   1 5
(2) INFORMATION FOR SEQ ID NO:27:
   (i) SEQUENCE CHARACTERISTICS:
      (A) LENGTH: 8 amino acids
      (B) TYPE: amino acid
      (D) TOPOLOGY: both
   (ii) MOLECULE TYPE: peptide
   (xi) SEQUENCE DESCRIPTION: SEQ ID NO:27:
   Cys Met Gly Arg Trp Arg Leu Cys
   1 5
(2) INFORMATION FOR SEQ ID NO:28:
   (i) SEQUENCE CHARACTERISTICS:
      (A) LENGTH: 8 amino acids
      (B) TYPE: amino acid
      (D) TOPOLOGY: both
   (ii) MOLECULE TYPE: peptide
   (xi) SEQUENCE DESCRIPTION: SEQ ID NO:28:
   Cys Val Gly Glu Cys Gly Gly Cys
   1 5
(2) INFORMATION FOR SEQ ID NO:29:
   (i) SEQUENCE CHARACTERISTICS:
      (A) LENGTH: 7 amino acids
      (B) TYPE: amino acid
      (D) TOPOLOGY: both
   (ii) MOLECULE TYPE: peptide
   (xi) SEQUENCE DESCRIPTION: SEQ ID NO:29:
   Cys Val Ala Trp Leu Asn Cys
   1 5
(2) INFORMATION FOR SEQ ID NO:30:
   (i) SEQUENCE CHARACTERISTICS:
      (A) LENGTH: 7 amino acids
      (B) TYPE: amino acid
      (D) TOPOLOGY: both
   (ii) MOLECULE TYPE: peptide
   (xi) SEQUENCE DESCRIPTION: SEQ ID NO:30:
   Cys Arg Arg Phe Gln Asp Cys
   1 5
(2) INFORMATION FOR SEQ ID NO:31:
   (i) SEQUENCE CHARACTERISTICS:
      (A) LENGTH: 7 amino acids
      (B) TYPE: amino acid
      (D) TOPOLOGY: both
   (ii) MOLECULE TYPE: peptide
   (xi) SEQUENCE DESCRIPTION: SEQ ID NO:31:
   Cys Leu Met Gly Val His Cys
   1 5
(2) INFORMATION FOR SEQ ID NO:32:
   (i) SEQUENCE CHARACTERISTICS:
      (A) LENGTH: 8 amino acide
      (B) TYPE: amino acid
      (D) TOPOLOGY: both
   (ii) MOLECULE TYPE: peptide
   (xi) SEQUENCE DESCRIPTION: SEQ ID NO:32:
   Cys Lys Leu Leu Ser Gly Val Cys
   1 5
(2) INFORMATION FOR SEQ ID NO:33:
   (i) SEQUENCE CHARACTERISTICS:
      (A) LENGTH: 8 amino acids
      (B) TYPE: amino acid
      (D) TOPOLOGY: both
   (ii) MOLECULE TYPE: peptide
   (xi) SEQUENCE DESCRIPTION: SEQ ID NO:33:
   Cys Phe Val Gly His Asp Leu Cys
   1 5
(2) INFORMATION FOR SEQ ID NO:34:
   (i) SEQUENCE CHARACTERISTICS:
      (A) LENGTH: 7 amino acids
      (B) TYPE: amino acid
      (D) TOPOLOGY: both
   (ii) MOLECULE TYPE: peptide
   (xi) SEQUENCE DESCRIPTION: SEQ ID NO:34:
   Cys Arg Cys Leu Asn Val Cys
   1               5
(2) INFORMATION FOR SEQ ID NO:35:
   (i) SEQUENCE CHARACTERISTICS:
      (A) LENGTH: 7 amino acids
      (B) TYPE: amino acid
      (D) TOPOLOGY: both
   (ii) MOLECULE TYPE: peptide
   (xi) SEQUENCE DESCRIPTION: SEQ ID NO:35.
   Cys Lys Leu Met Gly Glu Cys
   1               5
(2) INFORMATION FOR SEQ ID NO:36:
   (i) SEQUENCE CHARACTERISTICS:
      (A) LENGTH: 7 amino acids
      (B) TYPES amino acid
      (D) TOPOLOGY: both
   (ii) MOLECULE TYPE: peptide
   (xi) SEQUENCE DESCRIPTION: SEQ ID NO:36:
   Cys Xaa Xaa Xaa Xaa Xaa Cys
   1               5
(2) INFORMATION FOR SEQ ID NO:37:
   (i) SEQUENCE CHARACTERISTICS:
      (A) LENGTH: 8 amino acids
      (B) TYPE: amino acid
      (D) TOPOLOGY: both
   (ii) MOLECULE TYPE: peptide
   (xi) SEQUENCE DESCRIPTION: SEQ ID NO:37:
   Cys Xaa Xaa Xaa Xaa Xaa Xaa Cys
   1               5
(2) INFORMATION FOR SEQ ID NO:38:
   (i) SEQUENCE CHARACTERISTICS:
      (A) LENGTH: 9 amino acids
      (B) TYPE: amino acid
      (D) TOPOLOGY: both
   (ii) MOLECULE TYPE: peptide
   (xi) SEQUENCE DESCRIPTION: SEQ ID NO:38:
   Cys Xaa Xaa Xaa Xaa Xaa Xaa Xaa Cys
   1               5
(2) INFORMATION FOR SEQ ID NO:39:
   (i) SEQUENCE CHARACTERISTICS:
      (A) LENGTH: 10 amino acids
      (B) TYPE: amino acid
      (D) TOPOLOGY: unknown
   (ii) MOLECULE TYPE: peptide
   (xi) SEQUENCE DESCRIPTION: SEQ ID NO:39:
   Cys Xaa Xaa Xaa Xaa Xaa Xaa Xaa Xaa Xaa
   1               5                   10
(2) INFORMATION FOR SEQ ID NO:40:
   (i) SEQUENCE CHARACTERISTICS:
      (A) LENGTH: 20 amino acids
      (B) TYPE: amino acid
      (D) TOPOLOGY: both
   (ii) MOLECULE TYPE: peptide
   (xi) SEQUENCE DESCRIPTION: SEQ ID NO:40:
   Xaa Xaa Cys Xaa Xaa Xaa Xaa Xaa Xaa Xaa Xaa Xaa Xaa Xaa Xaa Xaa
   1               5                   10                  15
   Xaa Cys Xaa Xaa
               20
(2) INFORMATION FOR SEQ ID NO:41:
   (i) SEQUENCE CHARACTERISTICS:
      (A) LENGTH: 21 amino acids
      (B) TYPE: amino acid
      (D) TOPOLOGY: unknown
   (ii) MOLECULE TYPE: peptide
   (xi) SEQUENCE DESCRIPTION: SEQ ID NO:41:
   Xaa Xaa Cys Xaa Xaa Xaa Xaa Xaa Xaa Xaa Xaa Xaa Xaa Xaa Xaa Xaa
   1               5                   10                  15
   Xaa Xaa Xaa Xaa Xaa
               20
(2) INFORMATION FOR SEQ ID NO:42:
   (i) SEQUENCE CHARACTERISTICS:
      (A) LENGTH: 21 base pairs
      (B) TYPE: nucleic acid
      (C) STRANDEDNESS: single
      (D) TOPOLOGY: linear
   (xi) SEQUENCE DESCRIPTION: SEQ ID NO:42:
   TGTNNKNNKN NKNNKNNKTG T                                              21
(2) INFORMATION FOR SEQ ID NO:43:
   (i) SEQUENCE CHARACTERISTICS:
      (A) LENGTH: 9 amino acids
      (B) TYPE: amino acid
      (D) TOPOLOGY: both
   (ii) MOLECULE TYPE: peptide
   (xi) SEQUENCE DESCRIPTION: SEQ ID NO:43:
   Cys Val Arg Leu Asn Ser Leu Ala Cys
   1               5
(2) INFORMATION FOR SEQ ID NO:44:
   (i) SEQUENCE CHARACTERISTICS:
      (A) LENGTH: 4 amino acids
      (B) TYPE: amino acid
      (D) TOPOLOGY: unknown
   (ii) MOLECULE TYPE: peptide
   (xi) SEQUENCE DESCRIPTION: SEQ ID NO:44:
   Asp Xaa Xaa Arg
   1
(2) INFORMATION FOR SEQ ID NO:45:
   (i) SEQUENCE CHARACTERISTICS:
      (A) LENGTH: 4 amino acid
      (B) TYPE: amino acid
      (D) TOPOLOGY: unknown
   (ii) MOLECULE TYPE: peptide
   (ix) FEATURE:
      (A) MAME/KEY: Peptide
      (B) LOCATION: 1
      (D) OTHER INFORMATION: /note= "Xaa is about 1 to about 10 independently selected amino acids."
   (ix) FEATURE:
      (A) NAME/KEY: Peptide
      (B) LOCATION: 5
      (D) OTHER INFORMATION: /note= "Xaa is about 1 to about 10 independently selected amino acids."
   (xi) SEQUENCE DESCRIPTION: SEQ ID NO:45:
(2) INFORMATION FOR SEQ ID NO:46:
   (i) SEQUENCE CHARACTERISTICS:
      (A) LENGTH: 5 amino acids
      (B) TYPE: amino acid
      (D) TOPOLOGY: unknown
   (ii) MOLECULE TYPE: peptide
   (ix) FEATURE:
      (A) NAME/KEY: Peptide
      (B) LOCATION: 1
      (D) OTHER INFORMATION: /note= "Xaa is absent or is about 1 to about 10 independently selected amino acids."
   (ix) FEATURE:
      (A) NAME/KEY: Peptide
      (B) LOCATION: 5
      (D) OTHER INFORMATION: /note= "Xaa is about 1 to about 20 independently selected amino acids."
   (xi) SEQUENCE DESCRIPTION: SEQ ID NO:46:
   Xaa Val Leu Arg Xaa
   1 5
(2) INFORMATION FOR SEQ ID NO:47:
   (i) SEQUENCE CHARACTERISTICS:
      (A) LENGTH: 12 amino acids
      (B) TYPE: amino acid
      (D) TOPOLOGY: both
   (ii) MOLECULE TYPE: peptide
   (xi) SEQUENCE DESCRIPTION: SEQ ID NO:47:
   Cys Xaa Xaa Cys Xaa Xaa Xaa Cys Xaa Xaa Xaa Cys
   1 5 10
(2) INFORMATION FOR SEQ ID NO:48:
   (i) SEQUENCE CHARACTERISTICS:
      (A) LENGTH: 13 amino acids
      (B) TYPE: amino acid
      (D) TOPOLOGY: both
   (ii) MOLECULE TYPE: peptide
   (xi) SEQUENCE DESCRIPTION: SEQ ID NO:48:
   Cys Gly Arg Glu Cys Pro Arg Leu Cys Gln Ser Ser Cys
   1 5 10
(2) INFORMATION FOR SEQ ID NO:49:
   (i) SEQUENCE CHARACTERISTICS:
      (A) LENGTH: 13 amino acids
      (B) TYPE: amino acid
      (D) TOPOLOGY: both
   (ii) MOLECULE TYPE: peptide
   (xi) SEQUENCE DESCRIPTION: SEQ ID NO:49:
   Cys Gly Glu Ala Cys Gly Gly Gln Cys Ala Leu Pro Cys
   1 5 10
(2) INFORMATION FOR SEQ ID NO:50:
   (i) SEQUENCE CHARACTERISTICS:
      (A) LENGTH: 13 amino acids
      (B) TYPE: amino acid
      (D) TOPOLOGY: both
   (ii) MOLECULE TYPE: peptide
   (xi) SEQUENCE DESCRIPTION: SEQ ID NO:50:
   Cys Asn Gly Arg Cys Val Ser Gly Cys Ala Gly Arg Cys
   1               5                   10
(2) INFORMATION FOR SEQ ID NO:51:
   (i) SEQUENCE CHARACTERISTICS:
      (A) LENGTH: 13 amino acids
      (B) TYPE: amino acid
      (D) TOPOLOGY: both
   (ii) MOLECULE TYPE: peptide
   (xi) SEQUENCE DESCRIPTION: SEQ ID NO:51:
   Cys Gly Leu Met Cys Gln Gly Ala Cys Phe Asp Val Cys
   1               5                   10
(2) INFORMATION FOR SEQ ID NO:52:
   (i) SEQUENCE CHARACTERISTICS:
      (A) LENGTH: 6 amino acids
      (B) TYPE: amino acid
      (D) TOPOLOGY: Linear
   (ii) MOLECULE TYPE: peptide
   (xi) SEQUENCE DESCRIPTION: SEQ ID NO:52:
   Trp Gly Thr Gly Leu Cys
   1               5
(2) INFORMATION FOR SEQ ID NO:53:
   (i) SEQUENCE CHARACTERISTICS:
      (A) LENGTH: 7 amino acids
      (B) TYPE: amino acid
      (D) TOPOLOGY: both
   (ii) MOLECULE TYPE: peptide
   (xi) SEQUENCE DESCRIPTION: SEQ ID NO:53:
   Cys Ser Val Ala Asn Ser Cys
   1               5
(2) INFORMATION FOR SEQ ID NO:54:
   (i) SEQUENCE CHARACTERISTICS:
      (A) LENGTH: 7 amino acids
      (B) TYPE: amino acid
      (D) TOPOLOGY: both
   (ii) MOLECULE TYPE: peptide
   (xi) SEQUENCE DESCRIPTION: SEQ ID NO:54:
   Cys Ser Ser Thr Met Arg Cys
   1               5
(2) INFORMATION FOR SEQ ID NO:55:
   (i) SEQUENCE CHARACTERISTICS:
      (A) LENGTH: 8 amino acids
      (B) TYPE: amino acid
      (D) TOPOLOGY: both
   (ii) MOLECULE TYPE: peptide
   (xi) SEQUENCE DESCRIPTION: SEQ ID NO:55:
   Cys Leu Ser Gly Ser Leu Ser Cys
   1               5
(2) INFORMATION FOR SEQ ID NO:56:
   (i) SEQUENCE CHARACTERISTICS:
      (A) LENGTH: 9 amino acids
      (B) TYPE: amino acid
      (D) TOPOLOGY: both
   (ii) MOLECULE TYPE: peptide
   (xi) SEQUENCE DESCRIPTION: SEQ ID NO:56:
   Cys Ile Glu Gly Val Leu Gly Gly Cys
   1               5
(2) INFORMATION FOR SEQ ID NO:57:
   (i) SEQUENCE CHARACTERISTICS:
      (A) LENGTH: 9 amino acids
      (B) TYPE: amino acid
      (D) TOPOLOGY: both
   (ii) MOLECULE TYPE: peptide
   (xi) SEQUENCE DESCRIPTION: SEQ ID NO:57:
   Cys Asp Cys Arg Gly Asp Cys Phe Cys
   1 5
(2) INFORMATION FOR SEQ ID NO:58:
   (i) SEQUENCE CHARACTERISTICS:
      (A) LENGTH: 11 amino acids
      (B) TYPE: amino acid
      (D) TOPOLOGY: both
   (ii) MOLECULE TYPE: peptide
   (xi) SEQUENCE DESCRIPTION: SEQ ID NO:58:
   Ala Cys Asp Cys Arg Gly Asp Cys Phe Cys Gly
   1 5 10
(2) INFORMATION FOR SEQ ID NO:59:
   (i) SEQUENCE CHARACTERISTICS:
      (A) LENGTH: 6 amino acids
      (B) TYPE: amino acid
      (D) TOPOLOGY: linear
   (ii) MOLECULE TYPE: peptide
   (xi) SEQUENCE DESCRIPTION: SEQ ID NO:59:
   Gly Arg Gly Glu Ser Pro
   1 5

## Description

This invention was made with government support under CA 42507, CA 62042 and Cancer Center Support Grant CA 30199 awarded by the National Institutes of Health. The government has certain rights in this invention.

### BACKGROUND OF THE INVENTION

### FIELD OF THE INVENTION

The present invention relates generally to the fields of molecular medicine and drug delivery and, more specifically, to a method of *in vivo* panning for identifying a molecule that homes to a specific organ and to molecules that home to specific organs, including to normal organs and to tumors.

### BACKGROUND INFORMATION

Although the effect of a particular pathology often is manifest throughout the body of the afflicted person, generally, the underlying pathology may affect only a single organ or tissue. In many cases, drugs are the treatment of choice for a patient suffering a particular disease. It is rare, however, that a drug will target only the diseased tissue or organ. More commonly, drug treatment results in undesirable side effects due, for example, to generalized toxic effects throughout the patient's body. The nausea, loss of hair and drop in blood count that occur as a result of treating of a cancer patient with chemotherapeutic agents are examples of the undesirable side effects that can occur due to drug treatment.

The undesirable side effects that can occur when drugs are used to treat a disease most often are due to the inability of the drug to specifically target the diseased organ or tissue. For example, a cancer chemotherapeutic agent that targets rapidly proliferating cells would be useful to kill rapidly dividing cancer cells. However, such an agent also kills normal proliferating hematopoietic and epithelial cells. Thus, the dose of such a drug that can be administered to a patient is limited due to its toxic effect on normal cells.

Efforts have been made to increase the target specificity of various drugs. In some cases, a particular cell type present in a diseased tissue or organ may express a unique cell surface marker. In such a case, an antibody can be raised against the unique cell surface marker and a drug can be linked to the antibody. Upon administration of the drug/antibody complex to the patient, the binding of the antibody to the cell surface marker results in the delivery of a relatively high concentration of the drug to the diseased tissue or organ. Similar methods can be used where a particular cell type in the diseased organ expresses a unique cell surface receptor or a ligand for a particular receptor. In these cases, the drug can be linked to the specific ligand or to the receptor, respectively, thus providing a means to deliver a relatively high concentration of the drug to the diseased organ.

While linking a drug to a molecule that homes to a particular cell type present in a diseased organ or tissue provides significant advantages for treatment over the use of a drug, alone, use of this method is severely limited. In particular, very few cell type specific antibodies have been described and it can be difficult and time consuming to attempt to obtain an antibody that targets an organ in a particular patient suffering a pathology. Furthermore, few cell type specific surface markers have been described. Even where such markers have been described, the cells expressing the markers can be distributed among various tissues or organs, thereby limiting their usefulness as targets. Thus, it is important to identify specific target cell markers that are expressed in only one or a few tissues or organs and to identify molecules that specifically interact with such markers.

Various cell types can express unique markers and, therefore, provide potential targets for organ homing molecules. Endothelial cells, for example, which line the internal surfaces of blood vessels, can have distinct morphologies and biochemical markers in different tissues. The blood vessels of the lymphatic system, for example, express various adhesion proteins that serve to guide lymphocyte homing. In addition, endothelial cells present in lymph nodes express a cell surface marker that is a ligand for L-selectin and endothelial cells in Peyer's patch venules express a ligand for the α₄β₇ integrin. These ligands are involved in specific lymphocyte homing to their respective lymphoid organs. Thus, linking a drug to L-selectin or to the α₄β₇ integrin may provide a means for targeting the drug to diseased lymph nodes or Peyer's patches, respectively, provided that these molecules do not bind to similar ligands present in a significant number of other organs.

Although the homing molecules present in the blood vessels of non-lymphoid tissues have not been clearly defined, the ability of lymphocytes to return to the organ in which they were first stimulated indicates that organ-specific endothelial markers exist. Similarly, the homing or metastasis of particular types of tumor cells to specific organs provides further evidence that organ-specific markers exist. However, there remains a need to identify other organ-specific cell markers and the molecules that bind to them.

Methods are now available for producing large populations of molecules and for screening libraries of molecules to identify those of interest. For example, phage peptide display libraries can be used to express large numbers of peptides that can be screened *in vitro* with a particular target molecule or a cell of interest in order to identify peptides that specifically bind the target molecule or the cell. Screening of such phage display libraries has been used, for example, to identify ligands that specifically bind various antibodies and cell surface receptors.

Screening of a phage display library generally involves *in vitro* panning of the library using a purified target molecule. Phage that bind the target molecule can be recovered, individual phage can be cloned and the peptide expressed by a cloned phage can be determined. Such a peptide can be useful for delivery of a drug linked to the peptide to cells expressing the target molecule.

Unfortunately, very few target molecules that are expressed by only one or a few cell types have been identified. Furthermore, even where such a target molecule is known, it is uncertain whether a peptide that specifically binds the target molecule, as determined using an *in vitro* panning method, will bind to the target molecule *in vivo*. As a result, the identification of a peptide from a phage display library using an *in vitro* panning method essentially represents only a starting point for determining whether the identified peptide can be useful for an *in vivo* procedure. Thus, a need exists to develop *in vivo* methods for screening large numbers of molecules such as peptides in order to identify those that can home to one or more selected organs or tissues. The present invention satisfies this need and provides related advantages as well.

### SUMMARY OF THE INVENTION

The present invention provides a method of identifying a molecule that homes specifically to one or a few selected organs or tissues by using *in vivo* panning to screen a library of potential organ homing molecules. The *in vivo* panning method involves administering a library, which consists of a diverse population of molecules, to a subject and identifying molecules that home to one or more selected organs or tissues in the subject. The invention is exemplified by administration of a phage peptide display library to a subject and the identification of peptides expressed by phage that home to brain, to kidney or to a tumor such as a breast cancer or a melanoma in the subject. *In vivo* panning can be repeated one or more times until a molecule that homes to a selected organ or tissue with the desired selectivity is recovered.

The invention also provides brain homing peptides as defined in claim 9-15 having the motif, SRL (serine-arginine-lysine), such as the peptide CLSSRLDAC (SEQ ID NO: 3), or the motif VLR, such as the peptide WRCVLREGPAGGCAWFNRHRL (SEQ ID NO: 16). Such organ or tissue homing molecules are referred to herein, for convenience, as "organ homing" molecules. A "tumor homing molecule" is an example of an organ homing molecule. An organ homing molecule of the invention is useful, for example, for targeting a moiety such as a drug to the selected organ or tissue or for identifying the presence of a target molecule in a sample.

The invention further provides methods of identifying a target molecule by detecting selective binding of the target molecule to an organ homing molecule. For example, a peptide that selectively homes to a selected organ or tissue can be attached to a solid matrix for use in affinity chromatography. A sample of the selected organ or tissue can be obtained and passed over the affinity matrix under conditions that allow specific binding of the target molecule, which then can be collected and identified using well known biochemical methods. The target molecule can be useful, for example, for raising an antibody specific for the target molecule.

### DETAILED DESCRIPTION OF THE INVENTION

The present invention provides a method of identifying a molecule that homes specifically to one or a few selected organs or tissues, including to a tumor such as a melanoma or a breast cancer, by screening a library using *in vivo* panning (see Pasqualini and Ruoslahti, Nature 380:364-366 (1996). The identified organ homing molecules are useful, for example, for targeting a desired moiety such as a drug, a toxin or a detectable label, which can be linked to the molecule, to the selected organ or tissue. *In vivo* panning provides a direct means for identifying molecules that selectively home to a selected organ or tissue and, therefore, provides a significant advantage over previous methods, which require that a molecule identified using an *in* *vitro* screening method subsequently be examined to determine whether it maintains its specificity *in vivo*.

As used herein, the term "library" means a collection of molecules. A library can contain a few or a large number of different molecules, varying from about ten molecules to several billion molecules or more. If desired, a molecule can be linked to a tag, which can be a shared tag or a specific tag that can facilitate recovery or identification of the molecule.

As used herein, the term "molecule" is used broadly to mean an organic chemical such as a drug; a peptide, including a variant or modified peptide or peptide-like molecules such as a peptidomimetic or peptoid; or a protein such as an antibody or a growth factor receptor or a fragment thereof such as an Fv, Fd or Fab fragment of an antibody, which contains a binding domain. For convenience, the term "peptide" is used broadly herein to mean peptides, proteins, fragments of proteins and the like. A molecule can be a non-naturally occurring molecule, which does not occur in nature, but is produced as a result of *in vitro* methods, or can be a naturally occurring molecule such as a protein or fragment thereof expressed from a cDNA library.

Methods for preparing libraries containing diverse populations of various types of molecules such as peptides, peptoids and peptidomimetics are well known in the art and commercially available (see, for example, Ecker and Crooke, Biotechnology 13:351-360 (1995), and Blondelle et al., Trends Anal. Chem. 14:83-92 (1995), and the references cited therein ; see, also, Goodman and Ro, Peptidomimetics for Drug Design, in "Burger's Medicinal Chemistry and Drug Discovery" Vol. 1 (ed. M.E. Wolff; John Wiley & Sons 1995), pages 803-861, and Gordon et al., J. Med. Chem. 37:1385-1401 (1994)). Where a molecule is a peptide, protein or fragment thereof, the molecule can be produced *in vitro* directly or can be expressed from a nucleic acid, which is produced *in vitro.* Methods of synthetic peptide and nucleic acid chemistry are well known in the art.

A library of molecules also can be produced, for example, by constructing a cDNA expression library from mRNA collected from a cell, tissue, organ or organism of interest. Methods for producing such, libraries are well known in the art (see, for example, Sambrook et al., Molecular Cloning: A laboratory manual (Cold Spring Harbor Laboratory Press 1989)). Preferably, the peptide encoded by the cDNA is expressed on the surface of a cell or a virus containing the cDNA. For example, cDNA can be cloned into a phage vector such as fuse 5 (see Example I), wherein, upon expression, the encoded peptide is expressed as a fusion protein on the surface of the phage.

In addition, a library of molecules can comprise a library of nucleic acid molecules. Nucleic acid molecules that bind, for example, to a cell surface receptor are known in the art (see O'Connell et al., Proc. Natl. Acad. Sci., *USA* 93:5883-5887 (1996); Tuerk and Gold, Science 249:505-510 (1990); Gold et al., Ann. Rev. Biochem. 64:763-797 (1995)). Thus, a library of nucleic acid molecules can be administered to a subject having a tumor and tumor homing molecules can be identified by *in vivo* panning as disclosed herein. If desired, the nucleic acid molecules can be nucleic acid analogs that, for example, are less susceptible to attack by nucleases (see, for example, Jelinek et al., Biochemistry 34:11363-11372 (1995); Latham et al., Nucl. Acids Res. 22:2817-2822 (1994); Tam et al., Nucl. Acids Res. 22:977-986 (1994); Reed et al., Cancer Res. 59:6565-6570 (1990)).

As disclosed herein, *in vivo* panning comprises administering a library to a subject, collecting a sample of a selected organ or tissue and identifying an organ homing molecule. An organ homing molecule can be identified using various methods well known in the art. Generally, the presence of an organ homing molecule in a selected organ or tissue is identified based on one or more characteristics common to the molecules present in the library, then the structure of a particular organ homing molecule is identified. For example, a highly sensitive detection method such as mass spectrometry, either alone or in combination with a method such as gas chromatography, can be used to identify organ homing molecules in a selected organ or tissue. Thus, where a library comprises diverse molecules based generally on the structure of an organic molecule, the presence of an organ homing molecule such as a drug can be identified by detecting the presence of a parent peak for the particular molecule.

If desired, the sample of the selected organ or tissue can be collected, then processed using a method such as HPLC, which can provide a fraction enriched in molecules having a defined range of molecular weights or polar or nonpolar characteristics or the like. Conditions for HPLC will depend on the chemistry of the particular molecule and are well known to those skilled in the art. Similarly, methods for bulk removal of potentially interfering cellular materials such as DNA, RNA, proteins, lipids or carbohydrates are well known in the art as are methods for enriching a fraction containing an organic molecule using, for example, methods of selective extraction. For example, where a library comprises a population of diverse organic chemical molecules each linked to a specific oligonucleotide tag, such that the particular molecule is identified by determining the oligonucleotide sequence using the polymerase chain reaction (PCR), genomic DNA can be removed from the sample of the collected organ in order to reduce the potential for background PCR reactions. In addition, a library can comprise a population of diverse molecules such as organic chemical molecules each linked to a common, shared tag. Based on the presence and properties of the shared tag, molecules of the library that selectively home to an organ or tissue can be substantially isolated from a sample of the organ or tissue. These and other methods can be useful for enriching the sample of the collected organ for the particular organ homing molecule, thereby removing potentially contaminating materials from the collected organ sample and increasing the sensitivity of detecting the molecule.

Evidence provided herein indicates that a sufficient number of organ homing molecules selectively home to a selected organ or tissue during *in vivo* panning such that the molecules readily can be identified. For example, various independent phage expressing the same peptide were identified in brain (Table 1), in kidney (Table 2), and in a tumor formed from implanted breast carcinoma cells (Table 3) or from implanted melanoma cells (Table 4). Specifically, almost half of the kidney homing peptides that were sequenced had the amino acid sequence CLPVASC (SEQ ID NO: 21); two peptides that homed to brain constituted about 40% of the sequenced brain homing peptides; one peptide that homed to the breast tumor constituted about 43% of the sequenced breast tumor homing peptides; and two peptides that homed to the melanoma constituted greater than 75% of the sequenced melanoma homing peptides (see Examples II and III).

Although a substantial fraction of the identified organ homing molecules have the same structure, the peptide inserts of only a small number of isolated phage were determined. It should be recognized, however, that hundreds of thousands to millions of phage expressing organ homing peptides were recovered following various *in vivo* pannings for organ homing molecules. These results indicate that specific organ homing molecules will be present in substantial numbers in an organ following *in vivo* homing, thereby increasing the ease with which the molecules can be identified.

Ease of identification of an organ homing molecule, particularly an untagged molecule, depends on various factors, including the presence of potentially contaminating background cellular material. Thus, where the organ homing molecule is an untagged peptide, a larger number must home to the organ in order to identify the specific peptides against the background of cellular protein. In contrast, a much smaller number of an untagged organic chemical homing molecule is identifiable because such molecules normally are absent from or present in only small numbers in the body. In such a case, a highly sensitive method such as mass spectrometry can be used to identify an organ homing molecule. The skilled artisan will recognize that the method of identifying a molecule will depend, in part, on the chemistry of the particular molecule.

Where an organ homing molecule is a nucleic acid or is tagged with a nucleic acid molecule, an assay such as PCR can be particularly useful for identifying the presence of the molecule because, in principle, PCR can detect the presence of a single nucleic acid molecule (see, for example, Erlich, PCR Technology: Principles and Applications for DNA Amplification (Stockton Press 1989), which is incorporated herein by reference). Preliminary studies have demonstrated that, following intravenous injection of 10 ng of an approximately 6000 base pair plasmid into a mouse and 2 minutes in the circulation, the plasmid was detectable by PCR in a sample of lung. These results indicate that nucleic acids are sufficiently stable when administered into the circulation such that *in vivo* panning can be used to identify nucleic acid molecules that selectively home to a selected organ.

The molecules of a library can be tagged, which can facilitate recovery or identification of the molecule. As used herein, the term "tag" means a physical, chemical or biological moiety such as a plastic microbead, an oligonucleotide or a bacteriophage, respectively, that is linked to a molecule of the library. Methods for tagging a molecule are well known in the art (Hermanson, Bioconjugate Techniques (Academic Press 1996), which is incorporated herein by reference).

A tag, which can be a shared tag or a specific tag, can be useful for identifying the presence or structure of a molecule of a library. As used herein, the term "shared tag" means a physical, chemical or biological moiety that is common to each molecule in a library. Biotin, for example, can be a shared tag that is linked to each molecule in a library. A shared tag can be useful to identify the presence of a molecule of the library in a sample and also can be useful to substantially isolate the molecules from a sample. For example, where the shared tag is biotin, the biotin-tagged molecules in a library can be substantially isolated by binding to streptavidin or their presence can be identified by binding with a labeled streptavidin. Where a library is a phage display library, the phage that express the peptides are another example of a shared tag, since each peptide of the library is linked to a phage. In addition, a peptide such as the hemaglutinin antigen can be a shared tag that is linked to each molecule in a library, thereby allowing the use of an antibody specific for the hemaglutinin antigen to substantially isolate molecules of the library from a sample of a selected organ or tissue.

A shared tag also can be a nucleic acid sequence that can be useful to identify the presence of molecules of the library in a sample or to substantially isolate molecules of a library from a sample. For example, each of the molecules of a library can be linked to the same selected nucleotide sequence, which constitutes the shared tag. An affinity column containing a nucleotide sequence that is complementary to the shared tag then can be used to hybridize molecules of the library containing the shared tag, thus substantially isolating the molecules from an organ or tissue sample. A nucleotide sequence complementary to a portion of the shared nucleotide sequence tag also can be used as a PCR primer such that the presence of molecules containing the shared tag can be identified in a sample by PCR.

A tag also can be a specific tag. As used herein, the term "specific tag" means a physical, chemical or biological tag that is linked to a particular molecule in a library and is unique for that particular molecule. A specific tag is particularly useful if it is readily identifiable. A nucleotide sequence that is unique for a particular molecule of a library is an example of a specific tag. For example, the method of synthesizing peptides tagged with a unique nucleotide sequence provides a library of molecules, each containing a specific tag, such that upon determining the nucleotide sequence, the identity of the peptide is known (see Brenner and Lerner, Proc. Natl. Acad. Sci., *USA* 89:5381-5383 (1992), which is incorporated herein by reference). The use of a nucleotide sequence as a specific tag for a peptide or other type of molecule provides a simple means to identify the presence of the molecule in a sample because an extremely sensitive method such as PCR can be used to determine the nucleotide sequence of the specific tag, thereby identifying the sequence of the molecule linked thereto. Similarly, the nucleic acid sequence encoding a peptide expressed on a phage is another example of a specific tag, since sequencing of the specific tag identifies the amino acid sequence of the expressed peptide.

The presence of a shared tag or a specific tag can provide a means to identify or recover an organ homing molecule of the invention following *in vivo* panning. In addition, the combination of a shared tag and specific tag can be particularly useful for identifying an organ homing molecule. For example, a library of peptides can be prepared such that each is linked to a specific nucleotide sequence tag (see, for example, Brenner and Lerner, *supra*, 1992), wherein each specific nucleotide sequence tag has incorporated therein a shared tag such as biotin. Upon homing to an organ, the particular organ homing peptides can be substantially isolated from a sample of the organ based on the shared tag and the specific peptides can be identified, for example, by PCR of the specific tag (see Erlich, *supra*, 1989).

A tag also can serve as a support. As used herein, the term "support" means a tag having a defined surface to which a molecule can be attached. In general, a tag useful as a support is a shared tag. For example, a support can be a biological tag such as a virus or virus-like particle such as a bacteriophage ("phage"); a bacterium such as *E. coli*; or a eukaryotic cell such as a yeast, insect or mammalian cell; or can be a physical tag such as a liposome or a microbead, which can be composed of a plastic, agarose, gelatin or other material. If desired, a shared tag useful as a support can have linked thereto a specific tag. Thus, the phage display libraries used in the exemplified methods can be considered to consist of the phage, which is a shared tag that also is a support, and the nucleic acid sequence encoding the expressed peptide, the nucleic acid sequence being a specific tag.

In general, a support should have a diameter less than about 10 µm to about 50 µm in its shortest dimension, such that the support can pass relatively unhindered through the capillary beds present in the subject and not occlude circulation. In addition, a support can be nontoxic, so that it does not perturb the normal expression of cell surface molecules or normal physiology of the subject, and biodegradable, particularly where the subject used for *in vivo* panning is not sacrificed to collect a selected organ or tissue.

Where a molecule is linked to a support, the tagged molecule comprises the molecule attached to the surface of the support, such that the part of the molecule suspected of being able to interact with a target in a cell in the subject is positioned so as to be able to participate in the interaction. For example, where the molecule is suspected of being a β adrenergic agonist, the binding portion of the molecule attached to a support is positioned so it can interact with a β adrenergic receptor on a cell in the selected organ or tissue. Similarly, where the molecule is suspected of being a ligand for a growth factor receptor, the molecule is positioned on the support so that it can bind the receptor. If desired, an appropriate spacer molecule can be positioned between the molecule and the support such that the ability of the potential organ homing molecule to interact with the target molecule is not hindered. A spacer molecule also can contain a reactive group, which provides a convenient and efficient means of linking a molecule to a support and, if desired, can contain a tag, which can facilitate recovery or identification of the molecule (see Hermanson, *supra*, 1996).

As exemplified herein, a peptide suspected of being able to home to a selected organ or tissue such as brain was expressed as the N-terminus of a fusion protein, wherein the C-terminus consisted of a phage coat protein. Upon expression of the fusion protein, the C-terminal coat protein linked the fusion protein to the surface of a phage such that the N-terminal peptide was in a position to interact with a target molecule in an organ. Thus, a molecule having a shared tag was formed by the linking of a peptide to a phage, wherein the phage provided a biological support, the peptide molecule is linked as a fusion protein, the phage-encoded portion of the fusion protein acts as a spacer molecule, and the nucleic acid encoding the peptide provided a specific tag allowing identification of an organ homing peptide.

As used herein, the term "*in vivo* panning" means a method of screening a library by administering the library to a subject and identifying a molecule that selectively homes to one or a few selected organs or tissues. The term "administering to a subject", when used in referring to a library of molecules or a portion thereof, is used in its broadest sense to mean that the library is delivered to a selected organ or tissue, including, where desired, a tumor, such that the molecules of the library contact the selected organ or tissue.

A library can be administered to a subject, for example, by injecting the library into the circulation of the subject such that the molecules can pass through the selected organ or tissue; after an appropriate period of time, circulation is terminated by sacrificing the animal subject or by removing a sample of the organ (see Example I). Alternatively, a cannula can be inserted into a blood vessel in the subject, such that the library is administered by perfusion for an appropriate period of time, after which the library can be removed from the circulation through the cannula or the animal subject can be sacrificed or the organ can be sampled to terminate circulation. Similarly, a library can be shunted through one or a few organs by cannulation of the appropriate blood vessels in the subject. It is recognized that a library also can be administered to an isolated perfused organ. Such panning in an isolated perfused organ can be useful for identifying molecules that bind to the organ and, if desired, can be used as an initial screening of a library. For example, if a kidney homing molecule is desired, a library can be perfused through an isolated kidney, then the molecules that bound to the perfused kidney can be screened by *in vivo* panning to identify a kidney homing molecule.

The *in vivo* panning method is exemplified herein by screening a phage peptide display library in mice and identifying specific peptides that selectively home to brain, kidney or a tumor (see Examples II and III). However, phage libraries that display protein receptor molecules, including, for example, an antibody or an antigen binding fragment of an antibody such an Fv, Fd or Fab fragment; a hormone receptor such as a growth factor receptor; or a cell adhesion receptor such as an integrin or a selectin also can be used to practice the invention. Variants of such molecules can be constructed using well known methods such as random, site-directed or codon based mutagenesis (see Huse, U.S. Patent No. 5,264,563, issued November 23, 1993. Thus, various types of phage display libraries can be screened using the disclosed *in vivo* panning method.

Phage display technology provides a means for expressing a diverse population of random or selectively randomized peptides. Various methods of phage display and methods for producing diverse populations of peptides are well known in the art. For example, Ladner et al. (U.S. Patent No. 5,223,409, issued June 29, 1993) describe methods for preparing diverse populations of binding domains on the surface of a phage. In particular, Ladner et al. describe phage vectors useful for producing a phage display library, as well as methods for selecting potential binding domains and producing randomly or selectively mutated binding domains.

Similarly, Smith and Scott (Meth. Enzymol. 217:228-257 (1993); see, also, Scott and Smith, Science 249: 386-390 (1990) describe methods of producing phage peptide display libraries, including vectors and methods of diversifying the population of peptides that are expressed (see, also, Huse, WO 91/07141 and WO 91/07149 ; see, also, Example I). Phage display technology can be particularly powerful when used, for example, with a codon based mutagenesis method, which can be used to produce random peptides or randomly or desirably biased peptides (Huse, U.S. Patent No. 5,264,563, *supra*, 1993). These or other well known methods can be used to produce a phage display library, which can be subjected to the *in vivo* panning method of the invention in order to identify a peptide that homes to one or a few selected organs or tissues.

In addition, to screening a phage display library, *in vivo* panning can be used to screen various other types of libraries, including, for example, an RNA or cDNA library or a chemical library. If desired, the organ homing molecule can be tagged, which can facilitate recovery of the molecule from a selected organ or tissue or identification of the molecule in the organ or tissue. For example, where a library of organic molecules, each containing a shared tag, is screened, the tag can be a moiety such as biotin, which can be linked directly to the molecule or can be linked to a support containing the molecules. Biotin provides a shared tag useful for recovering the molecule from a selected organ or tissue using an avidin or streptavidin affinity matrix. In addition, a molecule or a support containing a molecule can be linked to a shared tag such as the hapten, 4-ethoxy-methylene-2-phenyl-2-oxazoline-5-one (phOx), which can be bound by an anti-phOx antibody linked to a magnetic bead as a means to recover the tagged molecule. Methods for purifying biotin or phOx tagged molecules are known in the art and the materials for performing these procedures are commercially available (e.g., Invitrogen; La Jolla CA; and Promega Corp.; Madison WI). In the case where a phage library is screened, the phage can be recovered using methods as disclosed in Example I.

*In vivo* panning provides a method for directly identifying molecules that can home to one or a few selected organs or tissues. As used herein, the term "home" or "selectively home" means that a particular molecule binds relatively specifically to a target molecule present in one or few selected organs or tissues following administration to a subject. In general, selective homing is characterized, in part, by detecting at least a two-fold (2x) greater specific binding of the molecule to the selected organ or tissue as compared to a control organ or tissue.

It should be recognized that, in some cases, a molecule can localize nonspecifically to an organ or tissue. For example, *in vivo* panning of a phage display library resulted in a large number of phage localized in organs such as liver, lung and spleen, which contain components of the reticuloendothelial system (RES). Selective homing in such tissues can be distinguished from nonspecific binding by detecting differences, for example, in the abilities of different individual phage to home to a selected organ or tissue, including an organ containing an RES component. For example, selective homing can be identified by combining a putative organ homing molecule such as a peptide expressed on a phage with a large excess of non-infective phage or with about a five-fold excess of phage expressing unselected peptides, injecting the mixture into a subject and collecting a sample of the selected organ or tissue. In the latter case, for example, provided the number of injected phage expressing organ homing peptides is sufficiently low so as to be nonsaturating for the target molecule, a determination that greater than 20% of the phage in the selected organ or tissue express the putative organ homing molecule is demonstrative evidence that the peptide expressed by the phage is a specific organ homing molecule. In addition, nonspecific localization can be distinguished from selective homing by performing competition experiments as described in Examples II.D. and IV.

Various methods can be useful for preventing nonspecific binding of a molecule to an organ containing a component of the RES. For example, a molecule that homes selectively to such an organ can be obtained by first blocking the RES using a material such as polystyrene latex particles or dextran sulfate (see Kalin et al., Nucl. Med. Biol. 20:171-174 (1993); Illum et al., J. Pharm. Sci. 75:16-22 (1986); Takeya et al., J. Gen. Microbiol. 100:373-379 (1977)); then administering the library to the subject. Thus, Illum et al. administered dextran sulfate 500 or polystyrene microspheres prior to administration of a test substance to block nonspecific uptake of the test substance by Kupffer cells, which are the RES component of the liver (Illum et al., *supra*, 1986). In addition, Takeya et al. reported that the RES in liver could be blocked using carbon particles, dextran sulfate 500 (DS-500) or silica (Takeya et al., *supra*, 1977). Also, Kalin et al. reported that blockage of the RES can be effected using a gelatine colloid (Kalin et al., *supra*, 1993). These and various other agents useful for blocking nonspecific uptake by the RES are known and routinely used.

Undesired binding of phage to RES or to other sites also can be prevented by coinjecting the mice with a specific phage display library together with the same phage made replication-deficient (see Smith et al., *supra*, 1990, 1993). In addition, a peptide that homes to an organ containing an RES component can be identified by preparing a phage display library using phage that exhibit low background binding to such organs. For example, Merrill et al. (Proc. Natl. Acad. Sci., *USA* 93:3188-3192 (1996), which is incorporated herein by reference) selected lambda-type phage that are not taken up by the RES and, as a result, remain in the circulation for a prolonged period of time. A filamentous phage variant can be selected using similar methods.

As disclosed herein, uptake of peptide-displaying phage by the RES in liver, for example, was blocked by coadministration of non-infective phage, which are not amplifiable. Phage expressing a CX₉ library were injected into mice, either alone or with an excess of non-infective phage (see Example II.E.). Coadministration of non-infective phage with library phage substantially reduced the amount of phage recovered in liver and, to a lesser extent spleen, whereas little or no difference was observed in the number of phage recovered from brain, kidney or lung. These results indicate that uptake of phage by organs such as liver and spleen, which, at least in part can be nonspecific due to the RES component of these organs, can be blocked by coadministration of non-infective phage with a peptide-displaying phage library.

Further experiments were performed to determine whether the phage that were present in liver upon coadministration with non-infective phage had selectively homed to the liver. A phage library was coadministered with non-infective phage, then phage recovered from the liver after a first round of *in vivo* panning were amplified *in vitro* and used for a second round of *in vivo* panning (see Example II.E.). Phage were recovered from liver and from brain (control organ) and quantitated after each round of *in vivo* panning. More phage that homed to liver as compared to brain were recovered following the second round of *in vivo* panning and the population of phage recovered from the liver following the first round of *in vivo* panning was enriched with phage that selectively home to liver. These results demonstrate that the RES component of an organ such as liver can be blocked, thus allowing use of the *in vivo* panning method to identify a molecule that selectively homes to such an organ.

As was observed for liver, a high amount of phage localization also was observed in lung. However, uptake of phage by lung is not nonspecific due, for example, to uptake by alveolar phagocytes, which constitute an RES component in lung. Specifically, phage recovery essentially was the same regardless of whether a phage library was injected alone or was coadministered with non-infective phage (see Example II.E.). Thus, the high uptake of phage observed in the lung (Pasqualini and Ruoslahti, *supra*, 1996) likely is due to the large amount of vasculature in the lungs.

Selective homing can be demonstrated by determining the specificity of an organ homing molecule for the selected organ or tissue as compared to a control organ or tissue. For example, a ratio of brain:kidney homing of up to 9:1 was observed for brain homing peptides (i.e., 9x greater binding to the selected organ as compared to the control organ; see Example II.A.).

Selective homing also can be demonstrated by showing that molecules that home to a selected organ, as identified by one round of *in vivo* panning, are enriched for organ homing molecules in a subsequent round of *in vivo* panning. For example, phage expressing peptides that selectively home to brain were isolated by *in vivo* panning, then were subjected to additional rounds of *in vivo* panning. As demonstrated in Example II.A., phage recovered from brain after a first round of screening showed an 8x enrichment in homing to brain as compared to kidney following a second round of screening and a 13x enrichment following a third round of *in vivo* panning. When a peptide that selectively homes to brain was linked to a moiety, i.e., a red blood cell (RBC), the peptide/RBC complex selectively homed to brain (see Example II.D.).

Due to the conserved nature of cellular receptors and of ligands that bind a particular receptor, the skilled artisan would recognize that an organ homing molecule identified using, for example, *in vivo* panning in a mouse also would bind to the corresponding target molecule in the selected organ or tissue of a human or other species. For example, an RGD-containing peptide that can specifically bind to an integrin expressed by a cell in a human subject also can bind integrins expressed in a variety of species, including integrins expressed in mammalian cells such as murine and bovine cells as well as in cells of more evolutionarily distant species such as *Drosophila*. The ability of an organ homing molecule identified using *in vivo* panning in an experimental animal such as a mouse readily can be examined for the ability to bind to the corresponding organ or tissue in a human subject by demonstrating, for example, that the molecule also can bind specifically *in vitro* to a sample of the selected organ or tissue obtained from a human subject. Thus, routine methods can be used to confirm that an organ homing molecule identified using *in vivo* panning in an experimental animal also can bind an organ-specific target molecule in a human subject. Furthermore, such *in vitro* contacting of an organ homing molecule with a sample suspected of containing a selected organ or tissue can identify the presence of the selected organ or tissue in the sample.

Furthermore, with regard to tumor homing molecules, the skilled artisan would recognize that a tumor homing molecule identified using, for example, *in vivo* panning in a mouse having a murine tumor of a defined histological type such as a melanoma also would bind to the corresponding target molecule in a melanoma in a human or other mammalian species. In addition, a tumor homing molecule that binds a target molecule present in the vasculature in a tumor grown in a mouse likely also can bind to the corresponding target molecule in the vasculature of a tumor in a human or other mammalian subject. A tumor homing molecule identified using *in vivo* panning in an experimental animal readily can be examined for the ability to bind to a corresponding tumor in a human patient by demonstrating, for example, that the molecule also can bind specifically *in vitro* to a sample of the tumor obtained from the patient. Thus, routine methods can be used to confirm that a tumor homing molecule identified using *in vivo* panning in an experimental animal also can bind a target molecule in a human tumor.

The steps of administering the library to the subject, collecting a sample of a selected organ or tissue and identifying the molecules that home to the selected organ or tissue, comprise a single round of *in vivo* panning. Although not required, one or more additional rounds of *in vivo* panning generally are performed. Where an additional round of *in vivo* panning is performed, the molecules recovered from the selected organ or tissue in the previous round are administered to a subject, which can be the same subject used in the previous round, where only a part of the selected organ or tissue was collected.

By performing a second round of *in vivo* panning, the relative binding selectivity of the molecules recovered from the first round can be determined by administering the identified molecules to a subject, collecting a sample of the selected organ or tissue, and determining whether more phage is recovered from the organ or tissue as compared to the first round of panning. In addition, if desired, a control organ or tissue can be collected and used as a basis to compare the molecules recovered from the selected organ with those recovered from a control organ. An additional round of *in vivo* panning also can indicate whether the molecules identified from the initially selected organ or tissue also can home to additional organs or tissues, thus defining a family of selected organs or tissues. Additional rounds of panning can be performed as desired.

Ideally, no molecules are recovered from a control organ or tissue following a second or subsequent round of *in vivo* panning. Generally, however, a proportion of the molecules also will be present in a control organ or tissue. In this case, the ratio of molecules in the selected organ as compared to the control organ (selected:control) can be determined. As described above, for example, phage that homed to brain following a first round of *in vivo* panning demonstrated a 13x enrichment in homing to the selected organ as compared to the control organ, kidney, following two additional rounds of panning (Example II).

Additional rounds of *in vivo* panning can be used to determine whether a particular molecule homes only to the selected organ or tissue or can recognize a target on the selected organ that also is expressed in one or more other organs or tissues or is sufficiently similar to the target in the originally selected organ or tissue. Where a molecule is found to direct homing to organs or tissues in addition to the originally selected organ, the organs or tissues are considered to constitute a family of selected organs or tissues. Using the method of *in vivo* panning, molecules that home to only a single selected organ or tissue and molecules that home to a family of selected organs or tissues can be defined. Such identification is expedited by collecting various organs or tissues during subsequent rounds of *in vivo* panning.

As used herein, the term "selected organ or tissue" is used in its broadest sense to mean an organ or a tissue to which a molecule selectively homes. In addition, the term "organ or tissue" is used broadly to mean organ, tissue or cell type, including a cancer cell, in which case the selected organ or tissue can be a tumor such as a primary tumor or a metastatic lesion. In general, a selected organ or tissue contains a cell that expresses a particular target molecule such as a cell surface receptor to which an organ homing molecule can bind. By performing at least two rounds of *in vivo* panning, the selectivity of homing of the molecule to the selected organ or tissue can be determined (see Example II). As discussed above, however, in some cases an organ homing molecule can selectively home to more than one selected organ or tissue, in which case the molecule is considered to be able to selectively home to a family of selected organs or tissues.

The term "control organ or tissue" is used to mean an organ or tissue other than a selected organ. A control organ or tissue is characterized by the inability of an organ homing molecule to home to the control organ or tissue. A control organ or tissue is useful for identifying nonspecific binding of a molecule. A control organ or tissue can be collected, for example, to identify nonspecific binding of the molecule or to determine the selectivity of homing of the molecule (see Examples I and II). In addition, nonspecific binding can be identified by administering, for example, a control molecule, which is known not to home to an organ or tissue but is chemically similar to a potential organ homing molecule. Alternatively, where the administered molecules are linked to a support, administration of the supports, alone, can be used to identify nonspecific binding. For example, a phage that expresses the gene III protein, alone, but that does not contain a peptide fusion protein, can be screened by *in vivo* panning to determine the level of nonspecific binding of the phage support.

In some cases it can take several rounds of *in vivo* panning to identify a control organ or tissue, since a molecule that selectively homes to an originally selected organ also may have the ability to selectively home to additional organs or tissues, thus defining a family of selected organs or tissues. The ability of a molecule to home to one or to a family of selected organs or tissues allows for the preparation of panels of organ homing molecules, wherein individual molecules variously home to one selected organ or tissue or to any of a family of selected organs or tissues with variable selectivity.

In addition, it can be useful to determine whether a molecule that selectively homes to a particular type of tumor also selectively homes to the normal tissue from which the tumor is derived. It can be desirable, for example, to determine whether a molecule that homes to a breast tumor also homes to normal breast tissue. Thus, where *in vivo* panning is used to identify a tumor homing molecule, a sample of the normal tissue counterpart of the tumor can be examined to determine whether homing of the molecule is selective for a target molecule expressed only by the tumor cells or the vascular tissue present in the tumor or is selective for a target molecule normally expressed in the organ or tissue from which the tumor is derived.

In general, a library of molecules, which contains a diverse population of random or selectively randomized molecules of interest, is prepared, then administered to an animal subject. At a selected time after administration, the animal subject is sacrificed and a selected organ or tissue or part of the organ or tissue is collected such that the molecules present in the selected organ or tissue can be identified. For example, a mouse was injected with a phage peptide display library, then, after about 1 to 4 minutes, the mouse was anesthetized, frozen in liquid nitrogen to terminate circulation of the phage, a selected organ (brain or kidney; see Examples I and II) or tissue (breast tumor or melanoma; see Example III) was collected, phage present in the selected organ were recovered and peptides that selectively homed to the selected organ or tissue were identified.

In the examples provided, the animals were sacrificed to collect the selected organ or tissue. It should be recognized, however, that only a part of the selected organ need be collected to recover a molecule that homes to that organ. For example, a part of the selected organ or tissue can be collected by biopsy, such that a molecule such as a peptide expressed by a phage, can be administered to the same subject a second time or more, as desired. Where the molecule that is to be administered a second time to the same subject is linked, for example, to a support or to a tag, the support or tag must be nontoxic and should be biodegradable, so as not to interfere with subsequent rounds of screening.

*In vitro* screening of phage libraries previously has been used to identify peptides that bind to antibodies or to cell surface receptors (Smith and Scott, *supra*, 1993). For example, *in vitro* screening of phage peptide display libraries has been used to identify novel peptides that specifically bound to integrin adhesion receptors (Koivunen et al., J. Cell Biol. 124:373-380 (1994a)) and to the human urokinase receptor (Goodson et al., Proc. Natl. Acad. Sci., *USA* 91:7129-7133 (1994)). However, such *in vitro* studies provide no insight as to whether a peptide that can specifically bind to a selected receptor *in vitro* also will bind the receptor *in vivo* or whether the binding peptide or the receptor are unique to a specific organ in the body. Furthermore, the *in vitro* methods are performed using defined, well-characterized target molecules in an artificial system. For example, Goodson et al. utilized cells expressing a recombinant urokinase receptor. Thus, the *in vitro* methods require prior knowledge of the target molecule and yield little if any information regarding *in vivo* utility. In contrast, the *in vivo* panning method disclosed herein requires no prior knowledge or availability of a target molecule and, therefore, provides a significant advantage over previous methods by identifying molecules that selectively home *in vivo* and the target molecule present in an organ or tissue.

Although a mechanism by which the disclosed method of *in vivo* panning works has not been fully defined, one possibility is that a molecule such as a peptide expressed on a phage recognizes and binds to a target molecule present on endothelial cells lining the blood vessels in a selected organ. Evidence indicates, for example, that the vascular tissues in various organs differ from one another and that such differences can be involved in regulating cellular trafficking in the body. For example, lymphocytes home to lymph nodes or other lymphoid tissues due, in part, to the expression of specific "address" molecules by the endothelial cell in those tissues (Salmi et al., Proc. Natl. Acad. Sci., *USA* 89:11436-11440 (1992)). Similarly, various leukocytes can recognize sites of inflammation due, in part, to the expression of endothelial cell markers induced by inflammatory signals (see Butcher and Picker, Science 272:60-66 (1996); Springer, Cell 76:301-314 (1994)). In addition, the endothelium present in kidney glomeruli contains a binding protein for the atrial natriuretic peptide (see Lewicki and Protter, in "Hypertension, Pathophysiology, Diagnosis and Management" 2d ed (Raven Press 1995), chapter 61). Thus, endothelial cell markers provide a potential target for directing, for example, a drug to a particular organ in a subject.

In some cases, the metastasis of cancer cells to specific organs also can be due to recognition by the tumor cell of an organ specific marker, including organ specific endothelial cell markers (Fidler and Hart, Science 217:998-1003 (1982)). The pattern of metastasis of many cancers can be explained by assuming that circulating tumor cells are preferentially trapped in the first vascular bed encountered. Thus, the lungs and the liver are the most frequent sites of cancer metastasis. However, some cancers show patterns of metastasis that are not explained by circulatory routing. Metastasis of such cancers can be due, instead, to the presence of selectively expressed address molecules such as endothelial cell surface molecules expressed in the organ to which the cancer metastasizes (see Goetz et al., Intl. J. Canc. 65:192-199 (1996); Zhu et al., Proc. Natl. Acad. Sci., *USA* 88:9568-9572 (1991); Pauli et al., Canc. Metast. Rev. 9:175-189 (1990); Nicolson, Biochim. Biophys. Acta 948:175-224 (1988)). The identification of molecules that bind to such organ-specific endothelial cell markers can provide a means to prevent tumor cell metastasis to the particular organ.

Brain, kidney and two different experimental tumors were selected as target organs or tissues to identify phage expressing peptides that selectively home to these selected organs or tissues because the blood vessels in these organs have unique characteristics. For example, the blood vessels in the brain form the "blood-brain barrier" and express at least one specific antigen (Schlosshauer and Herzog, J. Cell Biol. 110:1261-1274 (1990)). In addition, the vasculature within a tumor generally undergoes active angiogenesis, resulting in the continual formation of new blood vessels to support the growing tumor. Such angiogenic blood vessels are distinguishable from mature vasculature in that angiogenic vasculature expresses unique endothelial cell surface markers, including the αᵥβ₃ integrin (Brooks, Cell 79:1157-1164 (1994)) and receptors for angiogenic growth factors (Mustonen and Alitalo, J. Cell Biol. 129:895-898 (1995); Lappi, Semin. Canc. Biol. 6, 279-288 (1995)). Moreover, tumor vasculature is histologically distinguishable from blood vessel in general in that tumor vasculature is fenestrated (Folkman, Nat. Med. 1:27-31 (1995); Rak et al., Anticancer Drugs 6:3-18 (1995)). Thus, the unique characteristics of tumor vasculature make it a particularly attractive target for determining whether a molecule that homes specifically to a tumor can be identified by *in vivo* panning. Such a tumor homing molecule can be useful for directing an agent such as a chemotherapeutic drug to a tumor, while reducing the likelihood the agent will have a toxic effect on normal, healthy organs or tissues. Moreover, a molecule that homes selectively to tumor vasculature also may have use in targeting other types of neovasculature such as that present in inflammatory, regenerating or wounded tissues.

Using *in vivo* panning, phage expressing various peptides that selectively homed to brain, to kidney, to a breast tumor or to a melanoma were identified (see Tables 1-4). Due to the large size of the phage (300 nm) and the short time the phage were allowed to circulate, it is unlikely that a substantial number of phage would have exited the circulatory system, particularly in the brain and kidney. Thus, the organ homing molecules that were identified likely homed to and bound primarily endothelial cell surface markers that are expressed in an organ-specific manner (see, for example, Springer, Cell 76:301-314 (1994)). These results indicate that *in vivo* panning can be used to identify and analyze endothelial cell specificities.

Immunohistochemical staining of brain following administration of phage displaying the brain homing peptide CLSSRLDAC (SEQ ID NO: 3) revealed staining within the brain capillaries. No apparent preference for homing of the phage to any particular region of the brain was observed (see Pasqualini and Ruoslahti, *supra*, 1995). Similarly, phage expressing the kidney homing peptide CLPVASC (SEQ ID NO: 21) localized in kidney capillaries, particularly in the glomeruli and between the tubules. Such an analysis is not possible using endothelial cells in culture because the cultured cells tend to lose their tissue-specific differences (Pauli and Lee, Lab. Invest. 58:379-387 (1988)).

Although the conditions under which the *in vivo* pannings were performed identified organ homing peptides that generally bind to endothelial cell markers, specific binding of phage expressing tumor homing peptides also was observed in tumor parenchyma, indicating that target molecules expressed on specific cells within an organ or tissue also can be identified (see Example III). These results indicate that phage expressing peptides can pass through or leak from the blood vessels in the tumor, possibly due to the fenestrated nature of the blood vessels. Various normal organs such as liver also contain vasculature that allows, for example, phage expressing peptides to contact certain parenchymal cells. Thus, *in vivo* panning can be useful for identifying molecules that home to particular cells or tissue types in an organ as well as to endothelial cells.

Phage peptide display libraries were constructed essentially as described Smith and Scott (*supra*, 1993; see, also, Koivunen et al., Biotechnology 13:265-270 (1995)). Oligonucleotides encoding peptides having substantially random amino acid sequences were synthesized based on an "NNK" codon, wherein "N" is A, T, C or G and "K" is G or T. "NNK" encodes 32 triplets, which encode the twenty amino acids and an amber STOP codon (Scott and Smith, *supra*, 1990). At least one codon encoding cysteine also was included in each oligonucleotide so that cyclic peptides could be formed through disulfide linkages (see Example I). The oligonucleotides were inserted in frame with the sequence encoding the gene III protein (gIII) in the vector fuse 5 such that a peptide-gIII fusion protein can be expressed. Following expression, the fusion protein is expressed on the surface of the phage containing the vector (Smith and Scott, *supra*, 1993; Koivunen et al., *supra*, 1994b).

Remarkably, following *in vivo* panning, the phage that selectively homed to brain, to kidney or to tumors displayed only a few different peptide sequences (see Tables 1-4 and Examples II and III). In some cases, peptides having the same amino acid sequence were encoded by phage having different oligonucleotide sequences encoding the peptide. Furthermore, a family of brain homing peptides was identified, wherein each peptide in the family contained the common amino acid motif, SRL (serine-arginine-leucine), but different flanking amino acid sequences (see Table 1; SEQ ID NOS: 1, 3 and 5). In addition, two different peptides displayed the motif VLR (valine-leucine-arginine; see Table 1, SEQ ID NOS: 4 and 16). These results demonstrate that it is the peptide displayed by the phage, rather than some incidental mutant property of the phage, that directs homing to the selected organ or tissue.

The sequences of the brain, kidney or tumor homing motifs do not reveal any significant similarities with known ligands for endothelial cell receptors nor do they resemble any sequences listed in various data banks. However, some of the brain-homing motifs share a similarity with integrin-binding sequences. For example, one brain homing peptide contained an RLD sequence, which is recognized by certain integrins (Altieri et al., J. Biol. Chem. 265:12119-12122 (1990); Koivunen et al., *supra*, 1994a), and the DXXR (SEQ ID NO: 44) motif in another peptide resembles the RGD, DGR, and NGR motifs that bind to certain integrins (Ruoslahti, J. Clin. Invest. 87:1-5 (1991); Koivunen et al., *supra*, 1994a).

*In vivo* panning also was used to identify molecules that selectively home to a tumor. Mice bearing a breast tumor were injected with a phage library and phage that homed to the breast tumor were recovered and the peptides for 14 phage were determined (see Example III.A.). The 14 phage expressed four different peptides, including the peptide CGRECPRLCQSSC (SEQ ID NO: 48), which was expressed by six of the 14 phage.

The general applicability of the *in vivo* panning method for identifying molecules that home to a tumor was examined by injecting mice bearing a syngeneic melanoma with phage expressing a diverse population of peptides (see Example III.B.). The B16 mouse melanoma model was selected for these studies because the tumors that form are highly vascularized and because the biology of this tumor line has been thoroughly characterized (see Miner et al., Cancer Res. 42:4631-4638 (1982)). Furthermore, because the B16 melanoma cells are of mouse origin, species differences between the host and the tumor cell donor will not affect, for example, the distribution of phage into the tumor as compared to into normal organs.

About three-fold enrichment of phage homing into the tumor relative to brain (control organ) was obtained. In particular, two peptides, WGTGLC (SEQ ID NO: 52) and CLSGSLSC (SEQ ID NO: 55), represented greater than 75% of the peptides expressed on phage that were recovered from the tumor and sequenced (see Example III.B.). Immunohistochemical staining of tumor and other organs using an anti-phage antibody demonstrated that the CLSGSLSC (SEQ ID NO: 50) expressing phage produced strong staining in the melanoma, but essentially no staining in skin or in kidney (control organs). The staining pattern generally followed the blood vessels within the melanoma, but was not strictly confined to the blood vessels. These results indicate that tumor homing molecules can be identified by *in vivo* panning and that such molecules can home to vascular tissue in the tumor as well as to tumor parenchyma, probably due to the fenestrated nature of the blood vessels permitting ready exit of the phage from the circulatory system.

The identification of brain homing peptides and breast tumor homing peptides that contain potential integrin binding motifs such as "RE" and "NGR" indicates that *in vivo* panning can be useful to identify integrin binding peptides (see Koivunen et al., *supra*, 1995). The αᵥβ₃ integrin, for example, is expressed in blood vessels undergoing angiogenesis but not in quiescent endothelial cells. Binding of a peptide or antibody antagonist to the αᵥβ₃ integrin induces apoptosis of the angiogenic vessels, possibly by interrupting a signal transduced into the proliferating endothelial cells as a result of αᵥβ₃ integrin binding to the extracellular matrix (Brooks et al., *supra*, 1994).

Since the αᵥβ₃ integrin is expressed by endothelial cells in angiogenic vasculature, experiments were performed to determine whether tumor vasculature that is undergoing angiogenesis can be targeted *in vivo* using methods as disclosed herein. Phage expressing the peptide, CDCRGDCFC (SEQ ID NO: 57; "RGD phage;" see, Koivunen et al., *supra*, 1995), which is known to bind to the αᵥβ₃ integrin, were injected into mice bearing tumors formed from human breast carcinoma cells, human melanoma cells or mouse melanoma cells (see Example IV). The RGD phage selectively homed to each of the tumors, whereas such homing did not occur with control phage. For example, in mice bearing tumors formed by implantation of human breast carcinoma cells, a twenty- to eighty-fold greater number of the RGD phage, as compared to unselected control phage, accumulated in the tumor.

Tissue staining for the phage showed accumulation of the RGD phage in the blood vessels within the tumor, whereas no staining was observed in brain or kidney (control organs). Specificity of tumor homing of the RGD phage was demonstrated by competition experiments, in which coinjection of the RGD-containing peptide greatly reduced tumor homing of the RGD phage, whereas coinjection of a non-RGD-containing control peptide had no effect on homing of the RGD phage (see Example IV). These results demonstrate that the α_{V}β₃ target molecule is expressed on the luminal surface of endothelial cells in a tumor and that a peptide that binds to an α_{V}-containing integrin can bind selectively to this integrin and, therefore, to vasculature undergoing angiogenesis. In view of these results, the skilled artisan would recognize that an RGD peptide or an antibody that binds an α_{V} integrin, such as the monoclonal antibody LM609, which binds an α_{V}β₃ integrin (see Brooks et al., *supra*, 1994), can be used to target an organ or tissue containing angiogenic vasculature, including a tumor containing angiogenic vasculature.

The ability of a molecule that homes to a particular tumor to selectively home to another tumor of the same or a similar histologic type can be determined using, for example, human tumors grown in nude mice or mouse tumors grown in syngeneic mice for these experiments. For example, various human breast cancer cell lines, including MDA-MB-435 breast carcinoma (Price et al., Cancer Res. 50:717-721 (1990)), SKBR-1-II and SK-BR-3 (Fogh et al., J. Natl. Cancer Inst. 59:221-226 (1975)), and mouse mammary tumor lines, including EMT6 (Rosen et al., Intl. J. Cancer 57:706-714 (1994)) and C3-L5 (Lala and Parhar, Intl. J. Cancer 54:677-684 (1993)), are readily available and commonly used as models for human breast cancer. Using such breast tumor models, for example, information relating to the specificity of an identified breast tumor homing molecule for diverse breast tumors can be obtained and molecules that home to a broad range of different breast tumors or provide the most favorable specificity profiles can be identified. In addition, such analyses can yield new information, for example, about tumor stroma, since stromal cell gene expression, like that of endothelial cells, can be modified by the tumor in ways that cannot be reproduced *in vitro*.

Selective homing of a molecule such as a peptide or protein to a selected organ or tissue can be due to specific recognition by the peptide of a particular cell target molecule such as a cell surface receptor present on a cell in the organ or tissue. Selectivity of homing is dependent on the particular target molecule being expressed on only one or a few different cell types, such that the molecule homes to only one or a few organs or tissues. As discussed above, the identified brain, kidney and, at least in part, tumor homing peptides likely are recognizing endothelial cell surface markers in the blood vessels present in these organs or tissues. However, most different cell types, particularly cell types that are unique to an organ or tissue, can express unique target molecules. Thus, in organs such as liver, spleen or lymph node, where blood circulates through sinusoids formed by the cells specific for the organ, *in vivo* panning can be useful for identifying molecules that home to the particular organ.

An organ homing molecule such as the peptides exemplified in Tables 1-4 can be used to direct a moiety to a selected organ or tissue by linking the moiety to the molecule. As used herein, the term "moiety" is used broadly to mean a physical, chemical, or biological material that is linked to an organ homing molecule. For example, a moiety can be an agent such as a drug or can be a chambered microdevice, which can contain an agent. In addition, a moiety can be a molecule such as a protein or nucleic acid, to which an organ homing molecule is grafted for the purpose of directing the protein or nucleic acid to a selected organ or tissue. For example, a peptide organ homing molecule can be expressed as a fusion protein with a desired protein such that the peptide directs the protein to a selected organ.

A moiety can be a detectable label such a radiolabel or can be a cytotoxic agent, including a toxin such as ricin or a drug such as a chemotherapeutic agent or can be a physical, chemical or biological material such as a liposome, microcapsule, micropump or other chambered microdevice, which can be used, for example, as a drug delivery system. Generally, such microdevices, should be nontoxic and, if desired, biodegradable. Various moieties, including microcapsules, which can contain an agent, and methods for linking a moiety or chambered microdevice to a molecule of the invention are well known in the art and commercially available (see, for example, "Remington's Pharmaceutical Sciences" 18th ed. (Mack Publishing Co. 1990), chapters 89-91; Harlow and Lane, Antibodies: A laboratory manual (Cold Spring Harbor Laboratory Press 1988), each of which is incorporated herein by reference; see, also, Hermanson, *supra*, 1996).

Linking of a moiety to an organ homing molecule for the purpose of directing homing of the moiety to the selected organ or tissue is exemplified by the linking of a brain homing peptide to a RBC, wherein the peptide directed homing of the RBC to brain (see Example II.D.). These results indicate that an organ homing molecule of the invention can be linked to other cell types or to a physical, chemical or biological delivery system such as a liposome or other encapsulating device, which can contain an agent such as drug, in order to direct the cell type or the delivery system to a selected organ or tissue. For example, a tumor homing molecule identified by *in vivo* panning can be linked to a white blood cell (WBC) such as a cytotoxic T cell or a killer cell, wherein upon administration of the tumor homing molecule/WBC complex, the molecule directs homing of the WBC to the tumor, where the WBC can exert its effector function. Similarly, an organ homing molecule can be linked to a liposome or to a microcapsule comprising, for example, a permeable or semipermeable membrane, wherein an agent such as a drug to be delivered to a selected organ or tissue is contained within the liposome or microcapsule.

In one embodiment, an organ homing molecule is linked to a moiety that is detectable external to the subject in order to perform an *in vivo* diagnostic imaging study. For example, *in vivo* imaging using a detectably labeled brain homing peptide can identify a region in the brain where circulation is occluded. For such studies, a gamma ray emitting radionuclide such as indium-111 or technitium-99 can be linked to a brain homing molecule and, following administration to a subject, can be detected using a solid scintillation detector. Alternatively, a positron emitting radionuclide such as carbon-11 or a paramagnetic spin label such as carbon-13 can be linked to the molecule and, following administration to a subject, the localization of the moiety/molecule can be detected using positron emission transaxial tomography or magnetic resonance imaging, respectively.

Such *in vivo* imaging methods also can be used to identify the presence of cancer in a subject by linking an appropriate moiety to a tumor homing molecule, which can recognize a unique target expressed by the tumor cells or by the blood vessels formed by angiogenesis in the tumor. Such methods can identify a primary tumor as well as a metastatic lesion, which may not be detectable using other methods. Having identified the presence of such cancer, in another embodiment of the invention, the tumor homing molecule can be linked to a cytotoxic agent such as ricin or a cancer chemotherapeutic agent in order to direct the moiety to the tumor or can be linked to a chambered microdevice, which can contain, for example, a chemotherapeutic drug or other cytotoxic agent. Such a method can allow selective killing of the tumor, while substantially sparing normal tissues.

When administered to a subject, the molecule/moiety complex is administered as a pharmaceutical composition containing, for example, the complex and a pharmaceutically acceptable carrier. Pharmaceutically acceptable carriers are well known in the art and include, for example, aqueous solutions such as water or physiologically buffered saline or other solvents or vehicles such as glycols, glycerol, oils such as olive oil or injectable organic esters.

A pharmaceutically acceptable carrier can contain physiologically acceptable compounds that act, for example, to stabilize or to increase the absorption of the complex. Such physiologically acceptable compounds include, for example, carbohydrates, such as glucose, sucrose or dextrans, antioxidants, such as ascorbic acid or glutathione, chelating agents, low molecular weight proteins or other stabilizers or excipients. One skilled in the art would know that the choice of a pharmaceutically acceptable carrier, including a physiologically acceptable compound, depends, for example, on the route of administration of the composition. The pharmaceutical composition also can contain an agent such as a cancer therapeutic agent.

One skilled in the art would know that a pharmaceutical composition containing an organ homing molecule can be administered to a subject by various routes including, for example, orally or parenterally, such as intravenously. The composition can be administered by injection or by intubation. The pharmaceutical composition also can be an organ homing molecule linked to liposomes or other polymer matrices, which can have incorporated therein, for example, a drug such as a chemotherapeutic agent (Gregoriadis, Liposome Technology, Vol. 1 (CRC Press, Boca Raton, FL 1984), which is incorporated herein by reference). Liposomes, for example, which consist of phospholipids or other lipids, are nontoxic, physiologically acceptable and metabolizable carriers that are relatively simple to make and administer.

For the diagnostic or therapeutic methods disclosed herein, an effective amount of the molecule/moiety complex must be administered to the subject. As used herein, the term "effective amount" means the amount of the complex that produces the desired effect. An effective amount often will depend on the moiety linked to the organ homing molecule. Thus, a lesser amount of a radiolabeled molecule can be required for imaging as compared to the amount of a drug/molecule complex administered for therapeutic purposes. An effective amount of a particular molecule/moiety for a specific purpose can be determined using methods well known to those in the art.

The route of administration of an organ homing molecule will depend, in part, on the chemical structure of the molecule. Peptides, for example, are not particularly useful when administered orally because they can be degraded in the digestive tract. However, methods for chemically modifying peptides to render them less susceptible to degradation by endogenous proteases or more absorbable through the alimentary tract are well known (see, for example, Blondelle et al., *supra*, 1995; Ecker and Crooke, *supra*, 1995; Goodman and Ro, *supra*, 1995). Such methods can be performed on peptides identified by *in vivo* panning. In addition, methods for preparing libraries of peptide analogs such as peptides containing D-amino acids; peptidomimetics consisting of organic molecules that mimic the structure of peptide; or peptoids such as vinylogous peptoids, are known in the art and can be used to identify molecules that home to a selected organ or tissue and are stable for oral administration.

Organ homing molecules obtained using the methods disclosed herein also can be useful for identifying the presence of a target molecule such as a cell surface receptor or a ligand for a receptor, which is recognized by the organ homing peptide, or for substantially isolating the target molecule. For example, an organ homing peptide can be linked to a solid support such as a chromatography matrix. The linked peptide then can be used for affinity chromatography by passing an appropriately processed sample of a selected organ or tissue over the column in order to bind a particular target molecule. The target molecule, which forms a complex with the organ homing molecule, then can be eluted from the column and collected in a substantially isolated form. The substantially isolated target molecule can be characterized using well known methods. An organ homing molecule also can be linked to a detectable moiety such as a radionuclide, a fluorescent molecule, an enzyme or biotin and can be used, for example, to screen a sample in order to detect the presence of the target molecule or to follow the target molecule during various isolation steps.

The methods of the present invention were used to identify peptides that selectively home to a selected organ or tissue. Thus, the present invention also provides brain homing peptides such as CNSRLHLRC (SEQ ID NO: 1), CENWWGDVC (SEQ ID NO: 2), WRCVLREGPAGGCAWFNRHRL (SEQ ID NO: 16) and others as shown in Table 1. Furthermore, Kidney homing peptides such as CLPVASC (SEQ ID NO: 21), CGAREMC (SEQ ID NO: 22) and others as shown in Table 2, tumor homing peptides (such as the breast tumor homing peptides CGRECPRLCQSSC (SEQ ID NO: 48) and others as shown in Table 3) and melanoma homing peptides (WGTGLC (SEQ ID NO: 52), CLSGSLSC (SEQ ID NO: 55) and others as shown in Table 4) are described.

It should be recognized that cysteine residues were included in the peptides such that cyclization of the peptides could be effected. In fact, the peptides containing at least two cysteine residues cyclize spontaneously. However, such cyclic peptides also can be active when present in a linear form (see, for example, Koivunen et al., J. Biol. Chem. 268:20205-20210 (1993)). Thus, in some cases one or both of the cysteine residues in a peptide can be deleted without significantly affecting the organ homing activity of a peptide of the invention. For example, a peptide having the sequence LSSRLDA (SEQ ID NO: 19; compare SEQ ID NO: 3) also can be a brain homing peptide. Similarly, the amino acid residues N-terminal and C-terminal to the first and last cysteine residues, respectively, in a peptide such as WRCVLREGPAGGCAWFNRHRL (SEQ ID NO: 16) can be dispensable without substantially altering brain homing activity of the peptide. Thus, a peptide having the sequence VLREGPAGG (SEQ ID NO: 20) also can be useful as a brain homing peptide. Methods for determining the necessity of a cysteine residue or of amino acid residues N-terminal or C-terminal to a cysteine residue for organ homing activity of a peptide of the invention are routine and well known in the art.

An organ homing peptide is useful, for example, for targeting a desired moiety to the selected organ or tissue as discussed above. In addition, an organ homing peptide can be used to identify the presence of a target molecule in a sample. As used herein, the term "sample" is used in its broadest sense to mean a cell, tissue, organ or portion thereof that is isolated from the body. A sample can be, for example, a histologic section or a specimen obtained by biopsy or cells that are placed in or adapted to tissue culture. If desired, a sample can be processed, for example, by homogenization, which can be an initial step for isolating the target molecule to which an organ homing molecule binds.

An organ homing peptide such as a brain homing peptide can be used to identify the target molecule expressed in brain. For example, a brain homing peptide can be attached to a matrix such as a chromatography matrix to produce a peptide affinity matrix. A homogenized sample of brain can be applied to the peptide-affinity matrix under conditions that allow specific binding of the brain homing peptide to the target molecule (see, for example, Deutshcer, Meth. Enzymol., Guide to Protein Purification (Academic Press, Inc., ed. M.P. Deutscher, 1990), Vol. 182 ; see, for example, pages 357-379). Unbound and nonspecifically bound material can be removed and the specifically bound brain-derived target molecule can be isolated in substantially purified form.

The disclosed *in vivo* panning method can be used to detect four different kinds of target molecules in tumors. First, because tumor vasculature undergoes active angiogenesis, target molecules that are characteristic of angiogenic vasculature, in general, or angiogenic tumor vasculature, in particular, can be identified. Second, vascular target molecules that are characteristic of the tissue of origin of the tumor can be identified. Third, target molecules that are expressed in the vasculature of a particular type of tumor can be identified. Fourth, tumor stroma or tumor cell target molecules can be identified due to the fenestrated nature of tumor vasculature, which allows the potential organ homing molecules to leave the circulation and contact the tumor parenchyma.

As an alternative to using an organ or tissue sample, extracts of cultured endothelial cells can be used as the starting material in order to enhance the concentration of the receptor in the sample. For example, brain-derived endothelial cell lines such as BEND cells (Montesano et al., Cell 62:435-445 (1990)) can be examined for the expression of a particular receptor for a brain homing peptide). The presence of the receptor can be established by using phage binding and cell attachment assays (see, for example, Barry et al., Nat. Med. 2:299-305 (1996).

A cell line expressing a particular receptor can be identified and surface iodination of the cells can be used to label the receptor. The cells then can be extracted with octylglucoside and the extract can be fractionated by affinity chromatography using a brain homing peptide (see Table 1) coupled to a matrix such as Sepharose™. Extracts prepared from HUVEC cells (human umbilical vein endothelial cells) can be used as a control for the brain-derived endothelial cells. The purified receptor can be microsequenced and antibodies can be prepared. If desired, oligonucleotide probes can be prepared and used to isolate cDNA clones encoding the receptor. Alternatively, an anti-receptor antibody can be used to isolate a cDNA clone from an expression library (see Argraves et al., J. Cell Biol. 105:1183-1190 (1987)).

As an alternative to isolating the receptor, a nucleic acid encoding the receptor can be isolated using a mammalian cell expression cloning system such as the COS cell system. An appropriate library can be prepared, for example, using mRNA from primary brain endothelial cells (Lathey et al., Virology 176:266-273 (1990)). The nucleic acids can be cloned into the pcDNAI vector (Invitrogen), for example. Cells expressing a cDNA for the receptor can be selected by binding to the brain homing peptide. Purified phage can be used as the carrier of the peptide and can be attached to magnetic beads coated, for example, with anti-M13 antibodies (Pharmacia). Cells that bind to the peptide coating can be recovered using a magnet and the plasmids can be isolated. The recovered plasmid preparations then can be divided into pools and examined in COS cell transfections. The procedure can be repeated until single plasmids are obtained that can confer upon the COS cells the ability to bind the brain homing peptide.

Some of the following examples are intended to illustrate but not limit the present invention, which is only defined by the subject-matter of the claims.

### EXAMPLE I

### IN VIVO PANNING

This example demonstrates methods for preparing a phage library and screening the library using *in vivo* panning to identify phage expressing peptides that home to a selected organ or tissue.

### A. Preparation of phage libraries:

Phage display libraries were constructed using the fuse 5 vector as described by Koivunen et al. (*supra*, 1995; see, also, Koivunen et al. Meth. Enzymol. 245:346-369 (1994b), which is incorporated herein by reference). Six libraries encoding peptides designated CX₅C (SEQ ID NO: 36), CX₆C (SEQ ID NO: 37), CX₇C (SEQ ID NO: 38), CX₉ (SEQ ID NO: 39), X₂CX₁₄CX₂ (SEQ ID NO: 40) and X₂CX₁₈ (SEQ ID NO: 41) were prepared, where "C" indicates cysteine and "X_{N}" indicates the given number of individually selected amino acids. These libraries can display cyclic peptides when at least two cysteine residues are present in the peptide.

Oligonucleotides were constructed such that "C" was encoded by the codon TGT and "X_{N}" was encoded by NNK, where "N" is equal molar mixtures of A, C, G and T, and where "K" is equal molar mixtures of G and T. Thus, the peptide represented by CX₅C (SEQ ID NO: 36) can be represented by an oligonucleotide having the sequence TGT(NNK)₅TGT (SEQ ID NO: 42). Oligonucleotides were made double stranded by 3 cycles of PCR amplification, purified and ligated to the nucleic acid encoding the gene III protein in the fuse 5 vector such that, upon expression, the peptide is present as a fusion protein at the N-terminus of the gene III protein.

The vectors were transfected by electroporation into MC1061 cells. Bacteria were cultured for 24 hr in the presence of 20 µg/ml tetracycline, then phage were collected from the supernatant by precipitation twice using polyethylene glycol. Each library contained about 5 x 10⁹ to 5 x 10¹⁴ transducing units (TU; individual recombinant phage).

### B. In vivo panning of phage:

A mixture of phage libraries containing 1 x 10¹⁴ TU was diluted in 200 µl DMEM and injected into the tail vein of anesthetized BALB\c mice (2 month old females; Jackson Laboratories; Bar Harbor ME); Avertin™ (0.015 ml/g) was used as anesthetic (see Pasqualini and Ruoslahti, *supra*, 1996). After 1-4 minutes, mice were snap frozen in liquid nitrogen. To recover the phage, carcasses were partially thawed at room temperature for 1 hr, organs were collected and weighed, then were ground in 1 ml DMEM-PI (DMEM containing protease inhibitors (PI); phenylmethylsulfonyl fluoride (PMSF; 1 mM), aprotinin (20 µg/ml), leupeptin (1 µg/ml)).

Organ samples were washed 3 times with ice cold DMEM-PI containing 1% bovine serum albumin (BSA), then directly incubated with 1 ml K91-kan bacteria for 1 hr. Ten ml NZY medium containing 0.2 µg/ml tetracycline (NZY/tet) was added to the bacterial culture, the mixture was incubated in a 37°C shaker for 1 hr, then 200 µl aliquots were plated in agar plates containing 40 µg/ml tetracycline (tet/agar).

Individual colonies containing phage recovered from brain or kidney were grown for 16 hr in 5 ml NZY/tet. The bacterial cultures obtained from the individual colonies were pooled and the phage were purified and re-injected into mice as described above for a second round of *in vivo* panning. A third round of panning also was performed. Phage DNA was purified from individual bacterial colonies obtained from the second and the third round of *in vivo* panning and the DNA sequences encoding the peptides expressed by selected phage were determined (see Koivunen et al., *supra*, 1994b).

### EXAMPLE II

### CHARACTERIZATION OF PEPTIDES THAT HOME TO A SELECTED ORGAN

This example demonstrates that an organ homing peptide of the invention selectively homes to a selected organ and that an organ homing peptide identified by *in vivo* panning can be used to direct a moiety to a selected organ.

### A. Brain is the selected organ

Three rounds of *in vivo* panning in mice were performed. Kidney was used as a control organ. Mice were injected with two different mixtures of phage libraries. The first mixture contained libraries encoding CX₉ (SEQ ID NO: 39), CX₅C (SEQ ID NO: 36), CX₆C (SEQ ID NO: 37) and CX₇C (SEQ ID NO: 38) peptides (CX₅₋₇/CX₉ mixture; SEQ ID NOS: 36-39). The second mixture contained libraries encoding X₂CX₁₄CX₂ (SEQ ID NO: 40) and X₂CX₁₈ (SEQ ID NO: 41) peptides (X₂CX₁₈/X₂CX₁₄CX₂ mixture; SEQ ID NOS: 40 and 41).

The phage library mixtures were administered to mice via tail vein injection. Phage input was 1 x 10¹⁶ TU of the CX₅₋₇/CX₉ (SEQ ID NOS: 36-39) mixture or 1 x 10¹⁴ TU of the X₂CX₁₈/X₂CX₁₄CX₂ (SEQ ID NOS: 40 and 41) mixture. Phage were recovered from the brains of the injected mice, the recovered phage were amplified *in vitro*, then a second and a third round of *in vivo* panning was performed. During the second and third rounds of panning, phage were recovered from brain and from kidney and the number of TU from each organ was compared. This comparison revealed that 6x more phage from the CX₅₋₇/CX₉ (SEQ ID NOS: 36-39) mixture bound to brain than to kidney in the second round and 13x more of the CX₅₋₇/CX₉ (SEQ ID NOS: 36-39) phage bound to brain than to kidney in the third round of panning. Following administration of the X₂CX₁₈/X₂CX₁₄CX₂ (SEQ ID NOS: 40 and 41) mixture, an 11x and 8x enrichment of phage homing to the brain as compared to kidney occurred during the second and third rounds of panning, respectively. Thus, substantial enrichment of phage binding to the brain was observed following the second and third rounds of *in vivo* panning.

The amino acid sequences were determined for the inserts present in 73 cloned phage that were recovered from brain during the second and third rounds of *in vivo* panning. Peptides containing an SRL motif predominated (36% of the clones sequenced; see SEQ ID NOS: 1, 3 and 5), followed by peptides containing the VLR motif (20.5% of the clones; see SEQ ID NOS: 4 and 16) and the peptide CENWWGDVC (SEQ ID NO: 2; 19% of the clones; see Table 1). Other peptides that occurred much less frequently, but were present more than once, included CGVRLGC (SEQ ID NO: 6), CKDWGRIC (SEQ ID NO: 7), CLDWGRIC (SEQ ID NO: 8) and CTRITESC (SEQ ID NO: 9). Eight other sequences appeared only one time each and were not characterized further. The SRL tripeptide motif was present in several different sequence contexts, indicating that the nucleic acids encoding the peptides were derived from a number of independent phage. These results indicate that the selection of the peptides containing the SRL motif represents the specific binding of several independent phage displaying peptides with the SRL sequence and is not an artifact due, for example, to phage amplification. In addition, in some cases, different phage expressed peptides that had the same amino acid sequence, but were encoded by oligonucleotides having different sequences, thus confirming that homing of a particular phage to an organ is due to the specific peptide expressed on the phage.

To determine the specificity of brain homing of the individual motifs identified, phage displaying the predominant motifs were amplified individually, diluted to the same input titer and administered to mice. Following administration, brain and kidney were removed and the number of TU of phage in each organ was determined. The enrichment ratio of phage recovered from the selected organ, brain, as compared to the control organ, kidney, revealed that phage displaying one of the four most recovered peptides, CNSRLHLRC (SEQ ID NO: 1), CENWWGDVC (SEQ ID NO: 2), WRCVLREGPAGGCAWFNRHRL (SEQ ID NO: 16), CLSSRLDAC (SEQ ID NO: 3), each selectively targeted the brain as compared to the kidney. Specifically, the ratio of selective homing (brain:kidney) was about 8 for CNSRLHLRC (SEQ ID NO: 1) and for CLSSRLDAC (SEQ ID NO: 3), 4 for CENWWGDVC (SEQ ID NO: 2) and 9 for WRCVLREGPAGGCAWFNRHRL (SEQ ID NO: 16).

**TABLE 1**

| PEPTIDES FROM PHAGE RECOVERED FROM BRAIN | |
|---|---|
| A.A. SEQUENCE | # PHAGE DISPLAYING SAME A.A. SEQUENCE (% PHAGE) |
| | |

| CX₅₋₇C/CX₉ (36-39)* library: | |
|---|---|
| CNSRLHLRC (1)* | 16 (21.9%) |
| CENWWGDVC (2) | 14 (19.2%) |
| CLSSRLDAC (3) | 6 (8.2%) |
| CVLRGGRC (4) | 5 (6.8%) |
| CNSRLQLRC (5) | 4 (5.5%) |
| CGVRLGC (6) | 3 (4.1%) |
| CKDWGRIC (7) | 2 (2.8%) |
| CLDWGRIC (8) | 2 (2.8%) |
| CTRITESC (9) | 2 (2.8%) |
| CETLPAC (10) | 1 (1.4%) |
| CRTGTLFC (11) | 1 (1.4%) |
| CGRSLDAC (12) | 1 (1.4%) |
| CRHWFDVVC (13) | 1 (1.4%) |
| CANAQSHC (14) | 1 (1.4%) |
| CGNPSYRC (15) | 1 (1.4%) |

| X₂CX₁₈/X₂CX₁₄CX₂ (SEQ ID NOS: 40 and 41) library: | |
|---|---|
| WRCVLREGPAGGCAWFNRHRL (16) | 10 (13.7%) |
| YPCGGEAVAGVSSVRTMCSE (17) | 1 (1.4%) |
| LNCDYQGTNPATSVSVPCTV (18) | 1 (1.4%) |

| | |
|---|---|
| * - numbers in parentheses indicate SEQ ID NO:. | |

Two control phage showed low binding to both brain and kidney. These results demonstrate that *in vivo* panning can be used to screen phage display libraries in order to identify phage expressing peptides that home to a selected organ.

### B. Kidney as the selected organ

The same methodology used to isolate phage expressing brain homing peptides was used to isolate phage expressing peptides that home to kidney. In these experiments, brain was used as the control organ. A mixture of the CX₅C (SEQ ID NO: 36) and the CX₆C (SEQ ID NO: 37) libraries was administered as described above. Homing of phage to the kidney was obtained and an approximately 3x to 7x enrichment of phage homing to kidney was observed following a second round of *in vivo* panning.

The amino acid sequences were determined for the inserts in 48 cloned phage that homed to kidney. The peptides expressed by these phage were represented by two predominant sequences, CLPVASC (SEQ ID NO: 21; 46% of the clones sequenced) and CGAREMC (SEQ ID NO: 22; 17% of the clones; see Table 2). In addition, the peptide CKGRSSAC (SEQ ID NO: 23) appeared three times and three other peptides were present twice each. Phage expressing a CLPVASC (SEQ ID NO: 21), CGAREMC (SEQ ID NO: 22) or CKGRSSAC (SEQ ID NO: 23) peptide each exhibited selective homing to kidney; the ratio of selective kidney:brain homing was 7 for CLPVASC (SEQ ID NO: 21), 3 for CGAREMC (SEQ ID NO: 22) and 2 for CKGRSSAC (SEQ ID NO: 23).

These results demonstrate that the *in vivo* panning method is a generally applicable method for screening a phage library to identify phage expressing peptides that home to a selected organ. Database

**TABLE 2**

| PEPTIDES FROM PHAGE RECOVERED FROM KIDNEY | |
|---|---|
| A.A. SEQUENCE | # PHAGE DISPLAYING SAME A.A. SEQUENCE (% PHAGE) |
| CLPVASC (21)* | 22 (45.8%) |
| CGAREMC (22) | 8 (16.7%) |
| CKGRSSAC (23) | 3 (6.2%) |
| CWARAQGC (24) | 2 (4.2%) |
| CLGRSSVC (25) | 2 (4.2%) |
| CTSPGGSC (26) | 2 (4.2%) |
| CMGRWRLC (27) | 1 (2.1%) |
| CVGECGGC (28) | 1 (2.1%) |
| CVAWLNC (29) | 1 (2.1%) |
| CRRFQDC (30) | 1 (2.1%) |
| CLMGVHC (31) | 1 (2.1%) |
| CKLLSGVC (32) | 1 (2.1%) |
| CFVGHDLC (33) | 1 (2.1%) |
| CRCLNVC (34) | 1 (2.1%) |
| CKLMGEC (35) | 1 (2.1%) |

| | |
|---|---|
| * - numbers in parentheses indicate SEQ ID NO:. | |

searches did not reveal any significant homology of the brain homing or kidney homing peptides to known ligands for endothelial cell receptors.

### C. Peptide homing is specific:

In order to confirm the specificity of a peptide for directing homing to a selected organ, peptide competition experiments were performed. The cyclic peptide, CLSSRLDAC (SEQ ID NO: 3), which is one of the brain homing peptides (see Table 1) was synthesized (Immunodynamics; La Jolla CA) and purified by HPLC. The effect on the homing of phage expressing CLSSRLDAC (SEQ ID NO: 3), CENWWGDVC (SEQ ID NO: 2) or WRCVLREGPAGGCAWFNRHRL (SEQ ID NO: 16) to the brain was examined in order to determine whether co-administration of the synthetic peptide affected homing of the phage.

Phage expressing CLSSRLDAC (SEQ ID NO: 3), CENWWGDVC (SEQ ID NO: 2) or WRCVLREGPAGGCAWFNRHRL (SEQ ID NO: 16) were titrated to the same concentration and 1 x 10⁸ TU was injected into mice alone, or with 100 µg purified synthetic cyclic CLSSRLDAC (SEQ ID NO: 3) peptide. The synthetic CLSSRLDAC (SEQ ID NO: 3) peptide inhibited the homing of phage expressing CLSSRLDAC (SEQ ID NO: 3) by about 60%. This result demonstrates that the homing of the phage to brain is specifically due to the expression on the phage of the CLSSRLDAC (SEQ ID NO: 3) peptide.

The synthetic CLSSRLDAC (SEQ ID NO: 3) peptide also inhibited homing of phage expressing the WRCVLREGPAGGCAWFNRHRL (SEQ ID NO: 16) peptide by about 60%, but did not affect homing of CENWWGDVC (SEQ ID NO: 2) phage. This result indicates that the CLSSRLDAC (SEQ ID NO: 3) and WRCVLREGPAGGCAWFNRHRL (SEQ ID NO: 16) peptides can recognize the same target molecule in brain, whereas the CENWWGDVC (SEQ ID NO: 2) peptide recognizes a different target.

### D. The brain homing peptide CLSSRLDAC (SEQ ID NO: 3) directs red blood cells to brain

The synthetic cyclic CLSSRLDAC (SEQ ID NO: 3) peptide (1 mg) was labeled with iodine-125 using the Bolton and Hunter reagent (Amersham; Arlington Heights IL). Labeled peptide was purified by reverse phase HPLC on Sep-Pak™ cartridges (Waters, Millipore Corp.; Milford PA) and the ¹²⁵I-peptide (200 µg) was coupled to 1 ml glutaraldehyde-stabilized sheep RBC (Sigma Chemical Co.; St. Louis MO) according to the manufacturer's instructions.

Fifty µl ¹²⁵I-peptide/RBC (200,000 cpm), alone or with 10 mM of unlabeled CLSSRLDAC (SEQ ID NO: 3), which is a brain homing peptide, or CVRLNSLAC (SEQ ID NO: 43), which has no brain homing activity, was injected into the tail vein. After 2 min, each mouse was perfused through the heart with 50 ml DMEM and the brain and kidney were removed and assayed for radioactivity.

Approximately twice as much of the CLSSRLDAC/RBC complex (SEQ ID NO: 3) homed to brain than to kidney. Coadministration of unlabeled CLSSRLDAC (SEQ ID NO: 3) with the complex essentially completely inhibited brain homing of the complex but had no effect on complex localizing to the kidney, indicating that localization of the complex in the kidney was non-specific. Coadministration of unlabeled CVRLNSLAC (SEQ ID NO: 43) had no effect on selective homing of the complex to brain and no effect on the non-specific localization of the complex in the kidney. These results demonstrate that an organ homing peptide identified using *in vivo* panning can be linked to a moiety such as a blood cell and selectively directs homing of the linked moiety to the selected organ.

### E. In vivo panning for molecules that selectively home to an organ containing a component of the reticuloendothelial system

This example demonstrates that enhanced uptake of peptide-displaying phage by the RES in liver and spleen can be blocked by coadministration of non-infective phage, thereby allowing identification of molecules that selectively home to an RES-containing organ.

Approximately 1 x 10⁸ TU of amplified phage expressing a CX₉ (SEQ ID NO: 39) library were injected, iv, into mice, either alone or with a 1000-fold excess of non-infective phage, which are not amplifiable. After 2 min, the mice were sacrificed and phage were rescued from liver, lung, kidney, brain and spleen, and the number of phage (TU) per gram (TU/g) of tissue recovered was determined.

Coadministration of non-infective phage with library phage substantially reduced the amount of phage recovered in liver from about 1300 TU/g to about 200 TU/g and, in spleen from about 300 TU/g to about 200 TU/g. In comparison, little or no difference was observed in the number of phage recovered from brain, kidney or lung when the phage library was administered alone or in combination with the non-infective phage. This result demonstrates that nonspecific uptake of phage by organs such as liver and spleen, which contain an RES component, can be blocked by coadministration of non-infective phage with a phage library.

These results also demonstrated that uptake of phage by the lung was selective. Specifically, recovery of phage from lung essentially identical regardless of whether the phage library was injected alone or was coadministered with non-infective phage. Thus, the high uptake of phage previously observed in the lung (Pasqualini and Ruoslahti, *supra*, 1996) likely is due to the large amount of vasculature in the lungs and is not, for example, due to uptake of the phage by alveolar phagocytes, which constitute an RES component in lung.

In order to demonstrate that the phage that homed to liver when coadministered with non-infective phage, in fact, selectively homed to liver, a phage library was coadministered with non-infective phage, then phage recovered from the liver after a first round of *in vivo* panning were amplified *in vitro* and used for a second round of *in vivo* panning. Phage were recovered from liver and from brain (control organ) and quantitated after each round of *in vivo* panning.

Following the second round of *in vivo* panning, more phage homed to liver (350 TU/g) as compared to brain (200 TU/g). In addition, about 150 TU phage/g liver were recovered after the first round, whereas about 350 TU phage/g liver were recovered after the second round, indicating that the population of phage recovered from the liver following the first round of *in vivo* panning was enriched with phage that selectively home to liver. These results demonstrate that the RES component of an organ such as liver can be blocked, thus allowing use of the *in vivo* panning method to identify a molecule that selectively homes to such an organ.

### EXAMPLE III

### CHARACTERIZATION OF PEPTIDES THAT HOME TO A SELECTED TUMOR TISSUE

This example demonstrates that *in vivo* panning can be used to identify peptides that home to a breast tumor or to a melanoma.

### A. Peptides that home to a human breast carcinoma tumor implanted into a nude mouse

Human 435 breast carcinoma cells (Price et al., Cancer Res. 50:717-721 (1990)) were inoculated into the mammary fat pad of nude mice. When the tumors attained a diameter of about 1 cm, either a phage targeting experiment was performed, in which phage expressing a specific peptide were administered to the tumor bearing mouse, or *in vivo* panning was performed.

The breast tumor bearing mice were injected with 1 x 10⁹ phage expressing a library of CX₃CX₃CX₃C (SEQ ID NO: 47) peptides, where X₃ indicates three groups of independently selected, random amino acids. The phage were allowed to circulate for 4 min, then the mice were anesthetized, snap frozen in liquid nitrogen while under anesthesia, and the tumor was removed. Phage were isolated from the tumor and subjected to two additional rounds of *in vivo* panning.

Following the third round of panning, phage were quantitated and the peptide sequences expressed by 14 different cloned phage were determined. The 14 cloned phage expressed 4 different peptides (Table 3). These results demonstrate that the *in vivo* panning method can identify molecules that selectively home to breast tumor tissue.

### B. Peptides that home to B16 mouse melanoma cells implanted into a mouse

The general applicability of the *in vivo* panning method to identify peptides that selectively home to a tumor was examined by performing *in vivo* panning in mice bearing an implanted mouse melanoma tumor.

**TABLE 3**

| PEPTIDES FROM PHAGE RECOVERED FROM HUMAN BREAST CANCER | | |
|---|---|---|
| SEQUENCE | # clones | % of total phage sequenced |
| CX₃CX₃CX₃C (47)* library | | |
| CGRECPRLCQSSC (48) | 6 | 42.9 |
| CGEACGGQCALPC (49) | 4 | 28.6 |
| CNGRCVSGCAGRC (50) | 3 | 21.4 |
| CGLMCQGACFDVC (51) | 1 | 7.1 |

| | | |
|---|---|---|
| * - numbers in parentheses indicate SEQ ID NO:. | | |

Mice bearing a melanoma were produced by implantation of B16B15b mouse melanoma cells, which produce highly vascularized tumors. B16B15b mouse melanoma cells were injected subcutaneously into the mammary fat pad of nude mice (2 months old) and tumors were allowed to grow until the diameter was about 1 cm. *In vivo* panning was performed as disclosed above. Approximately 1 x 10¹² transducing units of phage expressing the CX₅C (SEQ ID NO: 36), CX₆C (SEQ ID NO: 37) or CX₅C (SEQ ID NO: 38) library were injected, iv, and allowed to circulate for 4 min. Mice then were snap frozen in liquid nitrogen while under anesthesia, tumor tissue and brain (control organ) were removed, and phage were isolated as described above. Three rounds of *in vivo* panning were performed.

The amino acid sequences were determined for the inserts in 89 cloned phage recovered from the B16B15b tumors. The peptides expressed by these phage were represented by two predominant sequences, CLSGSLSC (SEQ ID NO: 55; 52% of the clones sequenced) and WGTGLC (SEQ ID NO: 52; 25% of the clones; see Table 4). Reinfection of phage expressing one of the selected peptides resulted in approximately three-fold enrichment of phage homing to the tumor relative to brain.

**TABLE 4**

| PEPTIDES FROM PHAGE RECOVERED FROM MOUSE B16B15b MELANOMA | | |
|---|---|---|
| SEQUENCE | # clones | % of total phage |
| CX₅C (36)* library sequenced | | |
| WGTGLC (52) | 22 | 25% |
| CSVANSC (53) | 3 | 3% |
| CSSTMRC (54) | 2 | 2% |
| CX₆C (37) library CLSGSLSC (55) | 46 | 52% |
| CX₇C (38) library CIEGVLGGC (56) | 2 | 2% |

| | | |
|---|---|---|
| * - numbers in parentheses indicate SEQ ID NO:. | | |

Localization of the phage expressing a tumor homing peptide in the mouse organs was examined by immunohistochemical staining of the tumor and various other tissues. 1 x 10⁹ pfu of a control (insertless) phage or a phage expressing the tumor homing peptide, CLSGSLSC (SEQ ID NO: 50), were injected, iv, into tumor bearing mice and allowed to circulate for 4 min. The mouse then was perfused with DMEM and various organs, including the tumor were removed and fixed in Bouin's solution. Histologic sections were prepared and stained using anti-M13 (phage) antibodies (see Pasqualini and Ruoslahti, *supra*, 1996).

Strong immunostaining was evident in the melanoma obtained from a mouse injected with phage expressing the CLSGSLSC (SEQ ID NO: 50) tumor homing peptide. Staining of the melanoma generally was localized to the blood vessels within the tumor, although some staining also was present in the tumor parenchyma. Essentially no staining was observed in a tumor obtained from a mouse injected with the insertless control phage or in skin or in kidney samples obtained from mice injected with either phage. However, immunostaining was detected in the liver sinusoids and in spleen, indicating that phage can be trapped nonspecifically in organs containing RES.

These results demonstrate that the *in vivo* panning method is a generally applicable method for screening a phage library to identify phage expressing peptides that home to a selected organ or tissue, including a tumor.

### EXAMPLE IV

### IN VIVO TARGETING OF A PHAGE EXPRESSING AN AN RGD PEPTIDE TO A TUMOR

Human 435 breast carcinoma cells were inoculated into the mammary fat pad of nude mice. When the tumors attained a diameter of about 1 cm, phage expressing a specific RGD-containing peptide were administered to the tumor bearing mouse. Similar results to those discussed below also were obtained with nude mice bearing tumors formed by implantation of human melanoma C8161 cells or by implantation of mouse B16 melanoma cells.

1 x 10⁹ phage expressing the RGD-containing peptide, CDCRGDCFC (SEQ ID NO: 57; see, Koivunen et al., *supra*, 1995) or control (insertless) phage were injected intravenously (iv) into the mice and allowed to circulate for 4 min. The mice then were snap frozen while under anesthesia, and various organs, including tumor, brain and kidney, were removed and the phage present in the organs was quantitated (see Pasqualini and Ruoslahti, *supra*, 1996).

Approximately 2-3 times more phage expressing the CDCRGDCFC (SEQ ID NO: 57) peptide were detected in the breast tumor as compared to brain and kidney, indicating the CDCRGDCFC (SEQ ID NO: 57; RGD phage) peptide resulted in selective homing of the phage to the breast tumor. In a parallel study, unselected phage, which express various, diverse peptides, were injected into tumor-bearing mice and various organs were examined for the presence of phage. Far more phage were present in kidney and, to a lesser extent, brain, as compared to the tumor. Thus, the 80-fold more RGD-expressing phage than unselected phage concentrated in the tumor. These results indicate that phage expressing the RGD-containing peptide home to a tumor, possibly due to the expression of the αᵥβ₃ integrin on blood vessels forming in the tumor.

Specificity of the breast tumor homing peptide was demonstrated by competition experiments, in which coinjection of 500 µg free peptide, ACDCRGDCFCG (SEQ ID NO: 58) with the phage expressing the tumor homing peptide reduced the amount of phage in the tumor by about tenfold, whereas coinjection with the inactive control peptide, GRGESP (SEQ ID NO: 59) essentially had no effect. Immunohistochemical staining for the phage that homed to the breast tumors showed accumulation of the RGD phage in the blood vessels within the tumor, whereas no staining was observed in brain or kidney (control organs). These results demonstrate that phage displaying a peptide that can bind to an integrin expressed on angiogenic vasculature can selectively home *in vivo* to an organ or tissue such as a tumor containing such vasculature.

Although the invention has been described with reference to the disclosed examples, it should be understood that the invention is limited only by the following claims.

## Claims

1. A method of obtaining a molecule that selectively homes to a selected organ or tissue, comprising the steps of:
a. administering to a subject a library of diverse molecules;
b. collecting a sample of the selected organ or tissue; and
c. identifying a molecule that homes to said selected organ or tissue.

2. The method of claim 1, wherein each of said diverse molecules is linked to a tag.

3. The method of claim 2, wherein said tag is a support.

4. The method of claim 1, wherein said library is a phage display library.

5. The method of claim 4, wherein said molecule is a peptide.

6. The method of claim 1, wherein said library is selected from the group consisting of a nucleic acid library and a chemical library.

7. The method of claim 1, wherein said selected organ or tissue is selected from the group consisting of brain and kidney.

8. The method of claim 1, wherein said selected organ or tissue is a tumor.

9. A molecule obtainable by the method of claims 1 to 8, which is a peptide that selectively homes to brain, said peptide having the amino acid sequence:
X₁SRLX₂ (SEQ ID NO: 45),

10. The peptide of claim 9, said peptide having the amino acid sequence CLSSRLDAC (SEQ ID NO: 3).

11. The peptide of claim 9, said peptide having an amino acid sequence selected from the group consisting of CNSRLHLRC (SEQ ID NO: 1) and CNSRLQLRC (SEQ ID NO: 5).

12. A molecule obtainable by the method of claims 1 to 8, which is a peptide that selectively homes to brain, said peptide having the amino acid sequence:
X₃VLRX₄ (SEQ ID NO: 46),
wherein X₃ is absent or is about 1 to 10 independently selected amino acids and X₄ is about 1 to about 20 independently selected amino acids.

13. The peptide of claim 12, said peptide having an amino acid sequence selected from the group consisting of CVLRGGRC (SEQ ID NO: 4) and WRCVLREGPAGGCAWFNRHRL (SEQ ID NO: 16).

14. A molecule obtainable by the method of claims 1 to 8, which is a peptide that selectively homes to brain, said peptide having an amino acid sequence selected from the group consisting of CENWWGDVC (SEQ ID NO: 2) and CGVRLGC (SEQ ID NO: 6).

15. A molecule obtainable by the method of claims 1 to 8, which is a peptide that selectively homes to brain, said peptide having an amino acid sequence selected from the group consisting of CKDWGRIC (SEQ ID NO: 7); CLDWGRIC (SEQ ID NO: 8); and CTRITESC (SEQ ID NO: 9).

16. An *in vitro* method of directing a moiety to a selected organ or tissue, comprising the steps of:
a. linking the moiety to a molecule of claims 9 to 15 to form a complex comprising said moiety and said molecule; and
b. contacting said complex with the selected organ or tissue, wherein said molecule of claims 9 to 15 selectively binds to the selected organ or tissue, thereby directing said moiety to said selected organ or tissue.

17. The method of claim 16, wherein said moiety comprises a detectable label.

18. The method of claim 16 or 17, wherein said moiety comprises a cytotoxic agent.

19. The method of claims 16 to 18, wherein said moiety is a chambered microdevice.

20. Use of a molecule according to claims 9 to 15 for preparing a pharmaceutical composition for directing a moiety to the brain.

21. Use of claim 20, wherein said moiety comprises a detectable label.

22. Use of claims 20 or 21, wherein said moiety comprises a cytotoxic agent.

23. Use of claims 20 to 22, wherein said moiety is a chambered microdevice.

24. A method of identifying the presence of a target molecule, which specifically binds a molecule that selectively homes to a selected organ or tissue, comprising the steps of:
a. obtaining a sample of an organ or tissue suspected of expressing the target molecule;
b. contacting said sample with a molecule of claims 9 to 15 or a molecule obtained by the method of claims 1 to 8 under conditions that allow specific binding of said molecule of claims 9 to 15 or a molecule obtained by the method of claims 1 to 8 with said target molecule; and
c. detecting specific binding of said molecule of claims 9 to 15 or a molecule obtained by the method of claims 1 to 8, said specific binding identifying the presence of the target molecule in said sample.

25. A method for isolating a target molecule, which specifically binds a molecule that selectively homes to a selected organ or tissue, comprising the steps of:
a. obtaining a sample of the selected organ or tissue, which expresses the target molecule;
b. contacting said sample with a molecule of claims 9 to 15 or a molecule obtained by the method of claims 1 to 8 under conditions that allow specific binding of said molecule with said target molecule to form a complex; and
c. substantially isolating said target molecule from said complex.

## Patentansprüche

1. Verfahren zum Erhalten eines Moleküls, das sich selektiv in einem ausgewählten Organ oder Gewebe einfindet, bei dem man:
a. einem Subjekt eine Bibliothek verschiedener Moleküle verabreicht,
b. eine Probe des ausgewählten Organs oder Gewebes gewinnt; und man
c. ein Molekül identifiziert, das sich in dem ausgewählten Organ oder Gewebe einfindet.

2. Verfahren nach Anspruch 1, bei dem jedes dieser verschiedenen Moleküle mit einer Markierung (tag) verknüpft ist.

3. Verfahren nach Anspruch 2, bei dem das tag ein Träger ist.

4. Verfahren nach Anspruch 1, bei dem die Bibliothek eine Phagen-Display-Bibliothek ist.

5. Verfahren nach Anspruch 4, bei dem das Molekül ein Peptid ist.

6. Verfahren nach Anspruch 1, bei dem die Bibliothek aus der Gruppe bestehend aus einer Nukleinsäure-Bibliothek und einer chemischen Bibliothek ausgewählt ist.

7. Verfahren nach Anspruch 1, bei dem das ausgewählte Organ oder Gewebe aus der Gruppe bestehend aus Gehirn und Niere ausgewählt ist.

8. Verfahren nach Anspruch 1, bei dem das ausgewählte Organ oder Gewebe ein Tumor ist.

9. Molekül, erhältlich nach dem Verfahren der Ansprüche 1 bis 8, welches ein Peptid ist, das sich selektiv im Gehirn einfindet, wobei das Peptid die Aminosäuresequenz
X₁SRLX₂ (SEQ ID NO: 45)
aufweist, wobei X₁ und X₂ jeweils 1 bis etwa 10 unabhängig voneinander ausgewählte Aminosäuren sind.

10. Peptid nach Anspruch 9, wobei das Peptid die Aminosäuresequenz CLSSRLDAC (SEQ ID NO: 3) aufweist.

11. Peptid nach Anspruch 9, wobei das Peptid eine Aminosäuresequenz aufweist, die aus der Gruppe bestehend aus CNSRLHLRC (SEQ ID NO: 1) und CNSRLQLRC (SEQ ID NO: 5) ausgewählt ist.

12. Molekül, erhältlich nach dem Verfahren der Ansprüche 1 bis 8, welches ein Peptid ist, das sich selektiv im Gehirn einfindet, wobei das Peptid die Aminosäuresequenz
X₃VLRX₄ (SEQ ID NO: 46)
aufweist, wobei X₃ nicht vorhanden ist oder etwa 1 bis 10 unabhängig voneinander ausgewählte Aminosäuren und X₄ etwa 1 bis etwa 20 unabhängig voneinander ausgewählte Aminosäuren sind.

13. Peptid nach Anspruch 12, wobei das Peptid eine Aminosäuresequenz aufweist, die aus der Gruppe bestehend aus CVLRGGRC (SEQ ID NO: 4) und WRCVLREGPAGGCAWFNRHRL (SEQ ID NO: 16) ausgewählt ist.

14. Molekül, erhältlich nach dem Verfahren der Ansprüche 1 bis 8, welches ein Peptid ist, das sich selektiv im Gehirn einfindet, wobei das Peptid eine Aminosäuresequenz besitzt, die aus der Gruppe bestehend aus CENWWGDVC (SEQ ID NO: 2) und CGVRLGC (SEQ ID NO: 6) ausgewählt ist.

15. Molekül, erhältlich durch das Verfahren nach den Ansprüchen 1 bis 8, welches ein Peptid ist, das sich selektiv in Gehirngewebe einfindet, wobei das Peptid eine Aminosäuresequenz aufweist, die aus der Gruppe bestehend aus CKDWGRIC (SEQ ID NO: 7), CLDWGRIC (SEQ ID NO: 8) und CTRITESC (SEQ ID NO: 9) ausgewählt ist.

16. *In vitro*-Verfahren, mit dem eine Komponente zu einem ausgewählten Organ oder Gewebe dirigiert wird, bei dem man:
a. die Komponente mit einem Molekül der Ansprüche 9 bis 15 verknüpft, um einen Komplex zu bilden, der die Komponente und das Molekül umfaßt; und man
b. das ausgewählte Organ oder Gewebe mit dem Komplex kontaktiert, wobei das Molekül nach den Ansprüchen 9 bis 15 selektiv an das ausgewählte Organ oder Gewebe bindet, wodurch die Komponente zu dem ausgewählten Organ oder Gewebe dirigiert wird.

17. Verfahren nach Anspruch 16, bei dem die Komponente eine nachweisbare Markierung (label) enthält.

18. Verfahren nach Anspruch 16 oder 17, bei dem die Komponente ein zytotoxisches Agens umfaßt.

19. Verfahren nach den Ansprüchen 16 bis 18, bei dem die Komponente eine in Kammern aufgeteilte Mikrovorrichtung ist.

20. Verwendung eines Moleküls nach den Ansprüchen 9 bis 15 zur Herstellung einer pharmazeutischen Zusammensetzung, mittels derer eine Komponente zum Gehirn dirigiert werden kann.

21. Verfahren nach Anspruch 20, bei dem die Komponente eine nachweisbare Markierung umfaßt.

22. Verfahren nach Anspruch 20 oder 21, bei dem die Komponente ein zytotoxisches Agens umfaßt.

23. Verfahren nach den Ansprüchen 20 bis 22, bei dem die Komponente eine in Kammern aufgeteilte Mikrovorrichtung ist.

24. Verfahren zum Nachweis eines Zielmoleküls, welches spezifisch an ein Molekül bindet, das sich selektiv in einem ausgewählten Organ oder Gewebe einfindet, bei dem man:
a. eine Probe eines Organs oder Gewebes gewinnt, das im Verdacht steht ein derartiges Zielmolekül zu exprimieren;
b. die Probe mit einem Molekül nach den Ansprüchen 9 bis 15 oder einem nach dem Verfahren der Ansprüche 1 bis 8 erhaltenen Molekül unter Bedingungen kontaktiert, die eine spezifische Bindung des Moleküls nach den Ansprüchen 9 bis 15 oder eines nach dem Verfahren der Ansprüche 1 bis 8 erhaltenen Moleküls mit dem Zielmolekül ermöglichen; und man
c. spezifische Bindung des Moleküls nach den Ansprüchen 9 bis 15 oder eines nach dem Verfahren der Ansprüche 1 bis 8 erhaltenen Moleküls nachweist, wobei die spezifische Bindung auf das Vorhandensein eines Zielmoleküls in der Probe hinweist.

25. Verfahren zur Isolierung eines Zielmoleküls, das spezifisch an ein Molekül bindet, welches sich in einem ausgewählten Organ oder Gewebe einfindet, bei dem man:
a. eine Probe des ausgewählten Organs oder Gewebes gewinnt, das das Zielmolekül exprimiert;
b. die Probe mit einem Molekül nach den Ansprüchen 9 bis 15 oder einem nach dem Verfahren der Ansprüche 1 bis 8 erhaltenen Molekül unter Bedingungen kontaktiert, die eine spezifische Bindung des Moleküls mit dem Zielmolekül ermöglichen, um einen Komplex zu bilden; und
c. im wesentlichen das Zielmolekül aus dem Komplex isoliert.

## Revendications

1. Procédé pour obtenir une molécule sélectivement dirigée vers un organe ou un tissu sélectionné, comprenant les étapes consistant à :
a. administrer à un sujet une banque de molécules variées ;
b. récupérer un échantillon de l'organe ou du tissu sélectionné :
c. identifier une molécule qui est dirigée vers ledit organe ou tissu sélectionné.

2. Procédé selon la revendication 1, dans lequel chacune des dites diverses molécules est liée à une étiquette.

3. Procédé selon la revendication 2 dans lequel ladite étiquette est un support.

4. Procédé selon la revendication 1 dans lequel ladite banque est une banque dans un phage.

5. Procédé selon la revendication 4 dans lequel ladite molécule est un peptide

6. Procédé selon la revendication 1 dans lequel ladite banque est choisie au sein du groupe constitué par une banque d'acides nucléiques et une banque chimique.

7. Procédé selon la revendication 1 dans lequel ledit organe ou tissu sélectionné est choisi au sein du groupe constitué par le cerveau et le rein.

8. Procédé selon la revendication 1 dans lequel ledit organe ou tissu sélectionné est une tumeur.

9. Molécule susceptible d'être obtenue à l'aide de la méthode des revendications 1 à 8 qui est un peptide qui est sélectivement dirigé vers le cerveau, ledit peptide possédant la séquence d'acides aminés suivante :
X₁SRLX₂ (SEQ ID N°: 45)
dans laquelle X₁ et X₂ comprennent chacun de 1 à 10 acides aminés sélectionnés indépendamment.

10. Peptide de la revendication 9, ledit peptide ayant la séquence d'acides aminés CLSSRLDAC (SEQ ID N° : 3)

11. Peptide de la revendication 9, ledit peptide ayant une séquence d'acides aminés choisie au sein du groupe constitué par CNSRLHLRC (SEQ ID N° : 1) et CNSRLQLRC (SEQ ID N° : 5).

12. Molécule susceptible d'être obtenue à l'aide de la méthode des revendications 1 à 8 qui est un peptide qui est sélectivement dirigé vers le cerveau, ledit peptide possédant la séquence d'acides aminés suivante :
X₃VLRX₄ (SEQ ID N° : 46)
dans laquelle X₃ est absent ou constitué d'environ 1 à 10 acides aminés sélectionnés indépendamment et X₄ est constitué d'environ 20 acides aminés sélectionnés indépendamment.

13. Peptide de la revendication 12, possédant une séquence d'acides aminés choisie au sein du groupe constitué par CVLRGGRC (SEQ ID N° : 4) et WRCVLREGPAGGCAWFNRHRL (SEQ ID N° : 16).

14. Molécule susceptible d'être obtenue à l'aide de la méthode des revendications 1 à 8 qui est un peptide qui est sélectivement dirigé vers le cerveau, ledit peptide possédant une séquence d'acides aminés choisie au sein du groupe constitué par CENWWGDVC (SEQ ID N° : 2) et CGVRLGC (SEQ ID N° : 6).

15. Molécule susceptible d'être obtenue à l'aide de la méthode des revendications 1 à 8 qui est un peptide qui est sélectivement dirigé vers le cerveau, ledit peptide possédant une séquence d'acides aminés choisie au sein du groupe constitué par CKDWGRIC (SEQ ID N° : 7) ; CLDWGRIC (SEQ ID N° : 8) et CTRITESC (SEQ ID N° : 9).

16. Procédé *in vitro* pour diriger un résidu vers un organe ou un tissu sélectionné qui comprend les étapes consistant à :
a. lier le résidu à une molécule selon les revendications 9 à 15 pour former un complexe comprenant ledit résidu et ladite molécule ; et
b. mettre en contact ledit complexe avec l'organe ou le tissu sélectionné, ladite molécule selon les revendications 9 à 15 se liant sélectivement à un organe ou un tissu sélectionné, de manière à diriger ainsi ledit résidu vers ledit organe ou tissu sélectionné.

17. Procédé selon la revendication 16, dans lequel ledit résidu comprend un marqueur détectable.

18. Procédé selon la revendication 16 ou 17, dans lequel ledit résidu comprend un agent cytotoxique.

19. Procédé selon les revendications 16 à 18, dans lequel ledit résidu est un micro dispositif chambré.

20. Utilisation d'une molécule selon les revendications 9 à 15 pour préparer une composition pharmaceutique destinée à diriger un résidu vers le cerveau.

21. Utilisation selon la revendication 20, dans laquelle ledit résidu comprend un marqueur détectable.

22. Utilisation selon la revendication 20 ou 21, dans laquelle ledit résidu comprend un agent cytotoxique.

23. Utilisation selon les revendications 20 à 22, dans laquelle ledit résidu est un micro dispositif chambré.

24. Procédé d'identification de la présence d'une molécule cible, laquelle se lie de manière spécifique à une molécule sélectivement dirigée vers un organe ou un tissu sélectionné, comprenant les étapes consistant à :
a. obtenir un échantillon d'un organe ou d'un tissu suspecté d'exprimer la molécule cible;
b. mettre en contact ledit échantillon avec une molécule selon les revendications 9 à 15 ou une molécule obtenue à l'aide du procédé des revendications 1 à 8 dans des conditions qui permettent la liaison spécifique de ladite molécule selon les revendications 9 à 15 ou d'une molécule obtenue à l'aide du procédé des revendications 1 à 8, avec ladite molécule cible ;
c. détecter la liaison spécifique de ladite molécule selon les revendications 9 à 15 ou d'une molécule obtenue à l'aide du procédé des revendications 1 à 8, ladite liaison spécifique révélant la présence de la molécule cible dans ledit échantillon.

25. Procédé pour isoler une molécule cible qui se lie spécifiquement à une molécule sélectivement dirigés vers un organe ou un tissu sélectionné, comprenant les étapes consistant à :
a. obtenir un échantillon de l'organe ou du tissu sélectionné qui exprime la molécule cible;
b. mettre en contact ledit échantillon avec une molécule selon les revendications 9 à 15 ou une molécule obtenue à l'aide du procédé des revendications 1 à 8 dans des conditions qui permettent la liaison spécifique de ladite molécule avec ladite molécule cible pour former un complexe ; et
c. isoler de manière substantielle ladite molécule cible dudit complexe.
